# EUROPEAN PATENT SPECIFICATION

(11) **EP 0 843 018 B1**
(45) Date of publication and mention of the grant of the patent: **05.07.2000**
(21) Application number: 97308903.0
(22) Date of filing: 06.11.1997
(51) Int. Cl.: C12Q 1/02, G01N 33/50, G01N 33/68

(54) **Use of certain amides as probes for detection of antitubulin activity and resistance monitoring**
Verwendung bestimmter Verbindungen als Proben zum Nachweis der Antitubulinwirkung, sowie zur Überwachung der Resistenz
Utilisation de certains amides commes sondes pour la détection d'activité antitubuline et de surveillance de la résistance

(30) Priority: 14.11.1996 US 30920 P
(43) Date of publication of application: 20.05.1998
(73) Proprietor: ROHM AND HAAS COMPANY, Philadelphia, Pennsylvania 19106-2399 (US)
(72) Inventor: Young, David Hamilton, Ambler, Pennsylvania 19002 (US)
(74) Representative: Buckley, Guy Julian

(56) References cited:
- EP-A- 0 170 498
- WO-A-95/26505
- WO-A-96/11398
- ZAVALA F ET AL: "Structure-antitubulin activity relationships in steganacin congeners and analogs. Inhibition of tubulin polymerization in vitro by (.+-.)-isodeoxypodophyllotoxin" J. MED. CHEM. (JMCMAR,00222623);80; VOL.23 (5); PP.546-9, CNRS;INST. CHIM. SUBST. NAT.; GIF-SUR-YVETTE; FR., XP002055797
- CUSHMAN M ET AL: "Synthesis, Antitubulin and Antimitotic Activity, and Cytotoxicity of Analogs of 2-Methoxyestradiol, an Endogenous Mammalian Metabolite of Estradiol That Inhibits Tubulin Polymerization by Binding to the Colchicine Binding Site" J. MED. CHEM. (JMCMAR,00222623);95; VOL.38 (12); PP.2041-9, PURDUE UNIVERSITY;DEPARTMENT OF MEDICINAL CHEMISTRY; WEST LAFAYETTE; 47907; IN; USA (US), XP002055798
- BARBIER P ET AL: "Differential Effects of Ethyl 5-Amino-2-methyl-1,2-dihydro-3-phenyl pyrido[3,4-b]pyrazin-7-yl Carbamate Analogs Modified at Position C2 on Tubulin Polymerization, Binding, and Conformational Changes" BIOCHEMISTRY (BICHAW,00062960);95; VOL.34 (51); PP.16821-9, FACULTE PHARMACIE;MARSEILLE; F-13385; FR. (FR), XP002055799

## Description

Microtubules are intracellular filamentous structures present in all eukaryotic cells. As components of different organelles such as mitotic spindles, centrioles, basal bodies, cilia, flagella, axopodia and the cytoskeleton, microtubules are involved in many cellular functions including chromosome movement during mitosis, cell motility, organelle transport, cytokinesis, cell plate formation, maintenance of cell shape and orientation of cell microfibril deposition in developing plant cell walls. The major component of microtubules is tubulin, a protein composed of two subunits called alpha and beta. An important property of tubulin in cells is the ability to undergo polymerization to form microtubules or to depolymerize under appropriate conditions. This process can also occur *in vitro* using isolated tubulin.

Microtubules play a critical role in cell division as components of the mitotic spindle, an organelle which is involved in distributing chromosomes within the dividing cell precisely between the two daughter nuclei. Various drugs and pesticides prevent cell division by binding to tubulin or to microtubules. Anticancer drugs acting by this mechanism include the alkaloids vincristine and vinblastine, and the taxane-based compounds paclitaxel and docetaxel {see, for example, E.K. Rowinsky and R.C. Donehower, *Pharmacology and Therapeutics,* **52**, 35-84 (1991)}. Other antitubulin compounds active against mammalian cells include benzimidazoles such as nocodazole and natural products such as colchicine, podophyllotoxin and the combretastatins. Benzimidazole compounds which bind to tubulin are also widely used anthelmintics {McKellar, Q.A. and Scott, E.W., *J*. *Vet. Pharmacol. Ther.,* **13**, 223-247 (1990)}. Anti-tubulin herbicides are described in "The Biochemical Mode of Action of Pesticides", by J.R. Corbett, K. Wright and A.C. Baillie, pp. 202-223, and include dinitroanilines such as trifluralin, N-phenylcarbamates such as chlorpropham, amiprophos-methyl, and pronamide. Fungicides believed to act by binding to tubulin include zarilamide {Young, D. H. and Reitz, E.M., *Proceedings of the 10th International Symposium on Systemic Fungicides and Antifungal Compounds,* Reinhardsbrunn, ed by H. Lyr and C. Polter, 381-385, (1993)}, the benzimidazoles benomyl and carbendazim, and the N-phenylcarbamate diethofencarb {Davidse, L.C and Ishi, H. in "Modern Selective Fungicides", ed. by H. Lyr, 305-322 (1995)}.

Due to the success of tubulin as a biochemical target for drugs and pesticides, there is considerable interest in discovering new compounds which bind to tubulin. Various cell-free methods are available for detecting such compounds. A common method involves measuring the ability of test compounds to inhibit the polymerization of isolated tubulin into microtubules *in vitro* {see for example, E. Hamel, *Medicinal Research Reviews,* **16***,* 207-231 (1996)}. In a second method, interactions of test compounds with isolated tubulin can be detected in binding assays by measuring the ability of the test compound to influence binding of a second tubulin-binding ligand, used as a probe. Typically, the probe is radiolabeled to enable binding to be measured. A test compound which binds to tubulin may influence binding of the probe by binding to the same site site on the tubulin protein as the probe, and thus reduce the amount of probe which binds. Alternatively, binding may be influenced by means of an "allosteric" interaction in which the test compound binds to a different site from that of the probe and induces a conformational change in the tubulin protein which affects the binding site of the probe. Such an allosteric interaction may either increase or decrease binding of the probe. A third approach involves measuring the effect of test compounds on tubulin-associated guanosine triphosphatase activity {Duanmu, C., Shahrik, L. K., Ho, H. H. and Hamel, E., *Cancer Research,* **49***,* 1344-1348 (1989)}.

To screen large numbers of compounds by any of these methods is feasible at present only using tubulin from mammalian brain tissue, since it has not been possible to isolate sufficiently large amounts of purified tubulin from other sources. This limits the usefulness of these methods since many anti-tubulin compounds show great specificity with respect to their effects on microtubules from different sources. For example, the herbicides oryzalin and amiprophosmethyl inhibit the polymerization of plant tubulin but not brain tubulin, whereas colchicine is more than 100-fold more effective as an inhibitor of brain tubulin polymerization than of plant tubulin polymerization {Morejohn, L.C. and Fosket, D.E., 'Tubulin from plant tubulin polymerization {Morejohn, L.C. and Fosket, D.E., 'Tubulin from Plants, Fungi, and Protists', in "Cell and Molecular Biology of the Cytoskeleton", ed. by J.W. Shay, 257-329 (1986)}.

The present invention relates to the use of certain amide derivatives, known to inhibit the growth of eukaryotic cells, including fungal and plant cells {see, for example, US 3,661,991, 4,863,940 and 5,254,584), as probes in binding assays to screen compounds for antitubulin activity. EP 0 170 498 A is concerned with N-acetonyl-substituted-amide compounds, compositions containing them, and their use in combating phytopathogenic fungi. However, EP 0 170 498 A does not disclose or suggest the use of those amides for carrying out binding assays.

While radiolabeled probes such as colchicine {see for example, M. H. Zweig and C.F. Chignell, *Biochemical Pharmacology,* **22**, 2141-2150 (1973)} and vinblastine (see for example, R. Bai et al., *Journal of Biological Chemistry,* **265,** 17141 (1990)} have been used extensively in binding assays using isolated tubulin, these compounds bind noncovalently to tubulin. WO 95 26505 A is concerned with a solid phase immunoassay comprising contacting a tubulin protein or a tubulin-like peptide covalently linked to a suitable support with a solution containing a protease inhibitor and determining said inhibitor by using a monoclonal antibody which specifically recognizes the free end of the linked tubulin. WO 96 11398 A describes a method of determining tubulin protein polymerization activity in a test sample comprising combining the test sample with a polymerizable tubulin protein followed by contacting the resulting mixture with a material which traps polymerized tubulin protein. F. Zavala et al., *J. Med. Chem.* **1980**, 23, 546-549 describe the ability of analogues of the naturally occurring antitumor lignan steganacin to inhibit the microtubule assembly *in vitro.* M. Cushman et al., *J. Med. Chem.* **1995**, 38, 2041-2049 describe the synthesis of analogues of 2-methoxyestradiol and their ability to act as inhibitors of tubulin polymerization and colchicine binding using tubulin purified from bovine brain. P. Barbier et al., *Biochemistry* **1995**, *34*, 16821-16829 studied the potent antitubulin activity of the two chiral isomers of ethyl 5-amino-2-methyl-1,2-dihydro-3-phenylpyrido[3,4-b]pyrazin-7-yl carbamate. None of WO 95 26505 A, WO 96 11398 A, F. Zavala et al., M. Cushman et al. and P. Barbier et al. disclose or suggest the use of the amides of the present invention for such assays.

One advantage of the amide derivatives of this invention over existing antitubulin compounds in competitive binding assays results from their unique ability to bind covalently in a highly specific manner to tubulin, specifically to the beta-subunit of tubulin. In binding assays it is necessary to measure the amount of the probe which is bound to tubulin, and this generally involves separating the tubulin-bound probe from unbound probe. In the case of the amides, since binding is covalent the tubulin-bound probe is chemically stable allowing easy separation from the unbound probe by methods such as filtration or centrifugation. This enables their use not only in assays using isolated tubulin but also in assays using whole cells, crude cell extracts, and partially purified tubulin preparations, thus obviating the need for isolated tubulin and enabling tubulin-binding assays to be carried out in many different types of cell or cell extract. For example, test compounds which bind to plant or fungal tubulin may be detected in assays using plant or fungal cells or cell extracts and amide probes, providing suitable assays for screening large numbers of compounds. Amide probes may also be used to detect compounds which bind to tubulin in mammalian cells, providing a means of studying effects of test compounds on mammalian tubulin from sources other than brain. Typically, the effect of test compounds on the initial rate of binding of the amide probe is measured. An advantage of whole cell assays over cell-free assays the cell as well as its possible metabolic activation or inactivation, which may be important factors affecting biological activity.

A second aspect of the present invention involves the use of amide probes in binding assays to evaluate sensitivity of eukaryotic cells to pesticides or drugs which act by binding to tubulin. The use over a period of time of an antitubulin pesticide or drug can result in the development of cells which show reduced sensitivity (resistance) to the antitubulin compound. Resistance may be due to alterations in tubulin or may involve other mechanisms such as a change in the uptake or metabolism of the antitubulin compound. Resistance to antitubulin compounds due to alterations in tubulin have occurred in fungal pathogens {Davidse, L. C. and Ishi, H. in "Modern Selective Fungicides", ed. by H. Lyr, 305-322 (1995)}, algae {James, S. W. *et al., Journal of Cell* Science, **106**, 209-218 (1993)} and helminths {Beech, R. N. *et al.,* Genetics, **138**, 103-110 (1994)}. Resistant cells containing altered tubulin may show a difference in binding affinity for amides, allowing amide probes to be used in binding assays to detect such mutants. Such an assay can be carried out by comparing the rate of binding of an amide probe to cells or extracts of cells previously exposed to the antitubulin pesticide or drug with the rate of binding to untreated control cells or cell extracts.

For example, benzimidazole and thiophanate fungicides such as benomyl (methyl 1-(butylcarbamoyl)benzimidazol-2-ylcarbamate), fuberidazole (2-(2'-furyl)benzimidazole), thiabendazole (2-(4-thiazolyl)benzimidazole), carbendazim (methyl benzimidazol-2-ylcarbamate), thiophanate-methyl (1,2-bis(3-methoxycarbonyl-2-thioureido)benzene, and thiophanate (1,2-bis(3-ethoxycarbonyl-2-thioureido)benzene are known in the art for use against plant pathogenic fungi. However, the use of benzimidazole and thiophanate fungicides over a period of time can result in the development of fungal strains having reduced sensitivity to these fungicides, whereby the fungicides are much less effective in controlling a particular fungal disease. Such "resistant" fungi when isolated as pure cultures typically are from 10-fold to >1,000-fold less sensitive to benzimidazoles and thiophanates than fungi from locations which have not been exposed to these fungicides. Moreover, fungi which develop reduced sensitivity to one benzimidazole or thiophanate fungicide frequently also show reduced sensitivity to other benzimidazole or thiophanate fungicides. The N-phenylcarbamate fungicide diethofencarb is used commercially to control benzimidazole-resistant fungi such as *Botrytis cinerea.* However, its use has led to the development of fungal strains resistant to both benzimidazoles and diethofencarb. Current methods to detect fungal strains resistant to benzimidazoles, thiophanates or diethofencarb are labor-intensive and time-consuming. Some methods involve isolation of pure test cultures followed by *in vitro* assays of mycelial growth using fungicide-amended agar plates, or in vivo assays involving fungicide-treated leaves. Alternatively, slide germination tests of spores may be carried out in the presence of fungicide. Fungal strains which are resistant to diethofencarb and/or benzimidazoles and thiophanates typically contain altered tubulin proteins {see for example, Koenraadt, H. *et al., Phytopathology,* **82**, 1348-1354 (1992) and Yarden, O. and Katan, T., *Phytopathology,* **83**, 1478-1483 (1993)). Benzimidazole-resistant, diethofencarb-sensitive fungal strains typically show enhanced sensitivity to amide derivatives of the present invention, whereas benzimidazole-resistant, diethofencarb-resistant fungal strains typically show reduced sensitivity. While not wishing to be bound by theory, it is believed that amide probes can be used in binding assays to differentiate benzimidazole-resistant, diethofencarb-sensitive fungal strains which show enhanced ability to bind amide probes in assays using whole cells or cell extracts, or benzimidazole-resistant, diethofencarb-resistant fungal strains which show reduced ability to bind amide probes, from strains which are not resistant. Such assays may be less labor-intensive and time-consuming, and may also provide information as to whether the resistance mechanism involves a change in tubulin. Information about the mechanism of resistance may be useful in designing a resistance management strategy.

A third aspect of the present invention involves the use of amide probes in binding assays to detect and quantitate tubulin in cells or cell extracts. Tubulin is the subject of intense research due to its success as a target for drugs and pesticides and its important cellular functions. In such studies it is often desirable to detect and quantitate tubulin in cells or cell extracts. At present this is accomplished by various immunoassays {D. Thrower *et al.,* Methods in Cell Biology, vol. 37, pp. 129-145 (1993)}, sodium dodecyl sulfate polyacrylamide gel electrophoresis {B.M. Spiegelman *et al.,* Cell, vol. 12, pp. 587-600 (1977)}, binding to DEAE-cellulose {J.C. Bulinski *et al.,* Analytical Biochemistry, vol. 104, 432-439 (1980)), or by measuring colchicine-binding activity (Wilson, L., Biochemistry, vol. 9, pp. 4999-5007 (1970)}. Amide probes offer an alternative method to detect and quantitate tubulin based on measurement of amide-binding activity. Use of amide probes obviates the need for antibodies against tubulin, provides a simpler and more rapid method than either sodium dodecyl sulfate polyacrylamide gel electrophoresis or binding to DEAE-cellulose, and is applicable to measurement of tubulin levels in a variety of cells such as plant or fungal cells which are not sensitive to colchicine.

Various methods known to those with skill in the art can be used to detect the binding of amide probes to tubulin in assays using whole cells, crude cell extracts, partially purified tubulin preparations or isolated tubulin. This may, for example, involve the use of radiolabeled or fluorescent amide probes. Typically, the probe is incubated with cells, cell extracts or tubulin preparations and the amount of bound probe is determined following its separation from unbound probe by various separation techniques or a combination of such techniques. Separation techniques include, but are not limited to, centrifugation, chromatography, phase separation, precipitation of either the bound or unbound probe, or adhesion of either the bound or unbound probe to a solid substrate. Scintillation proximity assay technology (U.S. 4,568,649) provides another potential method to measure binding of the probe to tubulin. Yet another method involves detection of amide-bound tubulin, or amide-bound peptides released from tubulin by chemical or enzymatic treatment, by mass spectroscopy techniques.

Using certain amides that inhibit the growth of eukaryotic cells, the present invention provides methods for carrying out binding assays, which are useful for screening compounds for antitubulin activity, resistance monitoring and determining tubulin content, in eukaryotic cells, cell extracts and tubulin preparations, said amides having the structural formula 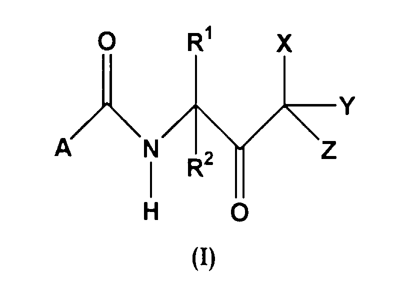 wherein
A is (C₃-C₇)cycloalkyl, (C₁-C₆)alkyl, halo(C₁-C₆)alkyl, (C₂-C₆)alkenyl, halo(C₂-C₆)alkenyl, (C₂-C₆)alkynyl, halo(C₂-C₆)alkynyl, phenyl, pyridyl, furyl, thienyl, isoxazolyl, oxazolyl, pyrrolyl, isothiazolyl, thiazolyl, pyrazolyl, imidazolyl, pyrimidinyl, quinolyl, isoquinolyl, naphthyl, pyridazinyl, pyrazinyl, benzothienyl, indolyl, benzofuranyl or benzyl; or phenyl, pyridyl, furyl, thienyl, isoxazolyl, oxazolyl, pyrrolyl, isothiazolyl, thiazolyl, pyrazolyl, imidazolyl, pyrimidinyl, quinolyl, isoquinolyl, naphthyl, pyridazinyl, pyrazinyl, benzothienyl, indolyl, benzofuranyl or benzyl substituted with four or less substituents each independently selected from halo, cyano, (C₁-C₆)alkyl, halo(C₁-C₆)alkyl, (C₂-C₆)alkenyl, halo(C₂-C₆)alkenyl, (C₂-C₆)alkynyl, halo(C₂-C₆)alkynyl, (C₁-C₆)alkoxy, halo(C₁-C₆)alkoxy, (C₁-C₆)alkylthio, halo(C₁-C₆)alkylthio, nitro, -NR⁶R⁷, -CR⁸=NOR⁹, -NHCOOR¹⁰, -CONR¹¹R¹², and -COOR¹³; or when A is an unsaturated ring and is substituted with two or more substituents which are adjacent to one another, two of said substituents may form a fused 5, 6 or 7 membered ring containing up to two heteroatoms selected from oxygen, nitrogen, sulfur and phosphorous;
R¹ and R² are each independently a hydrogen atom, (C₁-C₆)alkyl, halo(C₁-C₆)alkyl, (C₂-C₆)alkenyl, halo(C₂-C₆)alkenyl, (C₂-C₆)alkynyl or halo(C₂-C₆)alkynyl, provided that both R¹ and R² are not a hydrogen atom;
R⁶ and R⁷ are each independently a hydrogen atom, (C₁-C₆)alkyl or (C₁-C₆)alkylcarbonyl;
R⁸ is a hydrogen atom, (C₁-C₆)alkyl, (C₂-C₆)alkenyl or (C₂-C₆)alkynyl;
R⁹ is a hydrogen atom, (C₁-C₆)alkyl, (C₂-C₆)alkenyl, (C₂-C₆)alkynyl or (C₁-C₄)alkylcarbonyl;
R¹⁰, R¹¹, R¹² and R¹³ are each independently a hydrogen atom, (C₁-C₆)alkyl, (C₂-C₆)alkenyl or (C₂-C₆)alkynyl; and
X, Y and Z are each independently a hydrogen atom, halo, cyano, thiocyano, isothiocyano or (C₁-C₆)alkylsulfonyloxy, provided that at least one of X, Y and Z is not a hydrogen atom; or
the optical enantiomers thereof.

As used herein, "halo" includes fluoro, bromo, chloro, or iodo.

"Alkyl"includes straight or branched saturated hydrocarbon groups having from 1 to 6 carbons per group, for example, methyl, ethyl, *n*-propyl, isopropyl, *n*-butyl, isobutyl, *tert*-butyl, pentyl and hexyl.

"Alkenyl" includes straight or branched hydrocarbon groups having at least one double bond and from 2 to 6 carbons per group, for example, vinyl, 2-propenyl, 2-butenyl and 2-methyl-2-propenyl.

"Alkynyl" includes straight or branched hydrocarbon groups having at least one triple bond and from 2 to 6 carbons per group, for example, ethynyl, 2-propynyl and 2-butynyl.

"Alkoxy" includes straight or branched alkoxy groups having from 1 to 6 carbons per group, for example, methoxy, propoxy, *n*-butoxy and *tert*-butoxy.

"Alkylthio" includes straight or branched alkylthio groups having from 1 to 6 carbons per group, for example, methylthio and propylthio.

"Haloalkyl", "haloalkenyl", "haloalkynyl", "haloalkoxy" and "haloalkylthio" groups are "alkyl", "alkenyl", "alkynyl", "alkoxy" and "alkylthio" groups, respectively, which have from 1 to 5 halo substituents.

"Cycloalkyl" includes cyclic rings of from 3 to 7 carbon atoms, for example, cyclopropyl, cyclopentyl and cyclohexyl.

"Alkylcarbonyl" includes straight or branched alkyl groups having from 1 to 6 carbon atoms per group which are connected to a carbonyl group, for example, methylcarbonyl and butylcarbonyl.

"Alkylsulfonyloxy" includes straight or branched alkyl groups having from 1 to 6 carbon atoms per group which are connected to a sulfonyloxy group, for example, methylsulfonyloxy and propylsulfonyloxy.

Preferred methods of this invention use amides having the structural formula 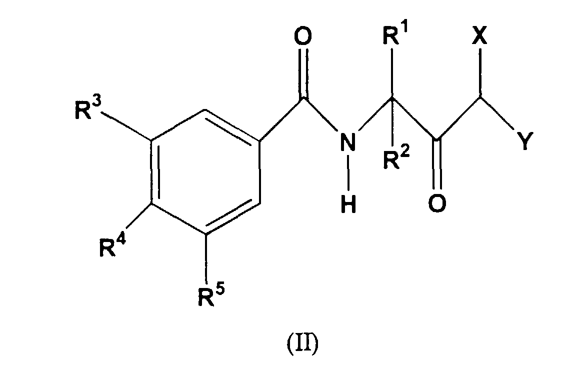 wherein
R¹ and R² are each independently a hydrogen atom, (C₁-C₆)alkyl, halo(C₁-C₆)alkyl, (C₂-C₆)alkenyl or (C₂-C₆)alkynyl, provided that both R¹ and R² are not a hydrogen atom;
R³, R⁴, and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, -NR⁶R⁷, -CR⁸=NOR⁹, -NHCOOR¹⁰, -CONR¹¹R¹² or -COOR¹³; or R⁴ and R⁵ taken together form a fused 5, 6, or 7-membered ring, which may contain up to two heteroatoms selected from the group consisting of O, S, N and P;
R⁶, R⁷, R⁸, R⁹, R¹⁰, R¹¹, R¹², and R¹³ are each independently a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

More preferred methods of this invention use benzoic acid amides having the structural formula (II) wherein
R¹ is methyl;
R² is methyl or ethyl;
R³ and R⁵ are each independently a hydrogen atom, halo, methyl, nitro, cyano, -NR⁶R⁷, -CR⁸=NOR⁹ or -NHCOOR¹⁰, provided that both R³ and R⁵ are not a hydrogen atom;
R⁴ is a hydrogen atom, halo, -NR⁶R⁷, cyano, -CR⁸=NOR⁹, -NHCOOR¹⁰, -COOR¹³ or (C₁-C₄)alkyl;
R⁶, R⁷, R⁸, R⁹, R¹⁰ and R¹³ are each independently a hydrogen atom or (C₁-C₆)alkyl;
X is chloro; and
Y is a hydrogen atom; or
the optical enantiomers thereof.

In assays employing mammalian cells, cell extracts or tubulin, even more preferred methods of this invention use benzoic acid amides having the structural formula (II) wherein
R¹ is methyl,
R² is ethyl,
R³ is halo or cyano,
R⁴ and R⁵ are amino or -CH=NOCH₃ provided that R⁴ and R⁵ are not the same,
X is chloro, and
Y is a hydrogen atom; or
the optical enantiomers thereof.

In assays employing fungal cells, cell extracts or tubulin in which the fungus belongs to the Ascomycete, Deuteromycete or Basidiomycete classes of fungi, even more preferred methods of this invention use benzoic acid amides having the structural formula (II) wherein
R¹ is methyl,
R² is methyl or ethyl,
R³ is -CH=NOCH₃,
R⁴ is a hydrogen atom, halo, amino, cyano or methyl,
R⁵ is halo, methyl, nitro or cyano,
X is chloro and
Y is a hydrogen atom; or
the optical enantiomers thereof.

In assays employing fungal cells, cell extracts or tubulin in which the fungus belongs to the Oomycete class of fungi, even more preferred methods of this invention use benzoic acid amides having the structural formula (II) wherein
R¹ is methyl,
R² is ethyl,
R³ and R⁵ are each independently a hydrogen atom, halo, methyl, nitro, cyano, -NR⁶R⁷, -CR⁸=NOR⁹ or -NHCOOR¹⁰, provided that both R³ and R⁵ are not a hydrogen atom,
R⁴ is a hydrogen atom, halo, -NR⁶R⁷, cyano, -CR⁸=NOR⁹, -NHCOOR¹⁰, -COOR¹³ or (C₁-C₄)alkyl,
R⁶, R⁷, R⁸, R⁹, R¹⁰ and R¹³ are each independently a hydrogen atom or methyl,
X is chloro, and
Y is a hydrogen atom; or
the optical enantiomers thereof.

In assays employing plant cells, cell extracts or tubulin, even more preferred methods of this invention use benzoic acid amides having the structural formula (II) wherein
R¹ is methyl,
R² is methyl or ethyl,
R³ and R⁵ are each independently a hydrogen atom, halo, methyl, nitro or cyano, provided that both R³ and R⁵ are not a hydrogen atom,
R⁴ is a hydrogen atom,
X is chloro, and
Y is a hydrogen atom; or
the optical enantiomers thereof.

Other preferred methods of this invention use pyridinecarboxamides having the structural formula 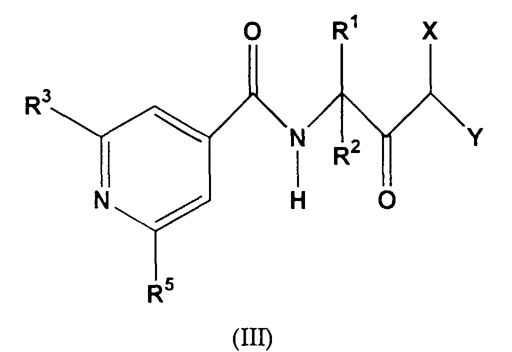 wherein
R¹ and R² are each independently a hydrogen atom, (C₁-C₆)alkyl, halo(C₁-C₆)alkyl, (C₂-C₆)alkenyl or (C₂-C₆)alkynyl, provided that both R¹ and R² are not a hydrogen atom;
R³ and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³;
R⁶, R⁷, R⁸, R⁹, R¹⁰, R¹¹, R¹², and R¹³ are a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

Yet other preferred methods of this invention use pyridinecarboxamides having the structural formula 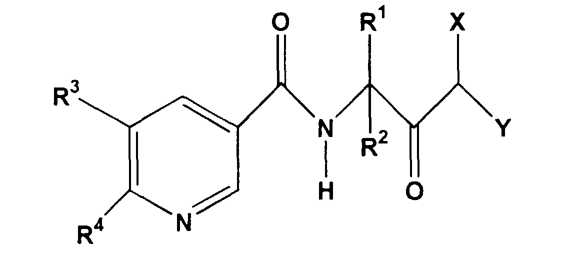 wherein
R¹ and R² are each independently a hydrogen atom, (C₁-C₆)alkyl, halo(C₁-C₆)alkyl, (C₂-C₆)alkenyl or (C₂-C₆)alkynyl, provided that both R¹ and R² are not a hydrogen atom;
R³ and R⁴ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³; or R³ and R⁴ taken together may form a fused 5, 6 or 7 membered ring which may contain up to two heteroatoms selected from the group consisting of: O, S, N, and P;
R⁶, R⁷, R⁸, R⁹, R¹⁰, R¹¹, R¹² and R¹³ are each independently a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

Other more preferred methods of this invention use pyridinecarboxamides having the structural formula (IV) wherein
R¹ is methyl;
R² is methyl or ethyl;
R³ and R⁴ are each independently a hydrogen atom, halo, cyano, methyl, nitro, -NR⁶R⁷, -CR⁸=NOR⁹ or -NHCOOR¹⁰, provided that both R³ and R⁴ are not a hydrogen atom;
R⁶, R⁷, R⁸, R⁹, R¹⁰ and R¹³ are each independently a hydrogen atom or (C₁-C₆)alkyl;
X is chloro and Y is a hydrogen atom; or
the optical enantiomers thereof.

Still other preferred methods of this invention use furylcarboxamides or thienylcarboxamides having the structural formula 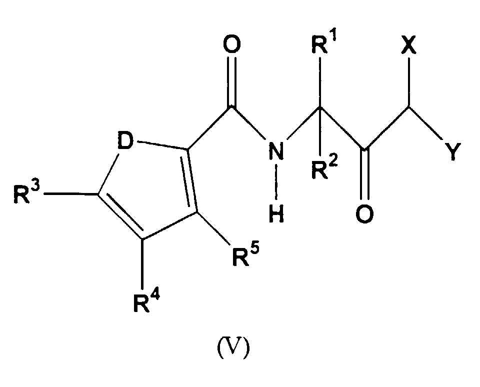 wherein
D is O or S;
R¹ and R² are each independently a hydrogen atom, (C₁-C₆)alkyl, halo(C₁-C₆)alkyl, (C₂-C₆)alkenyl, and (C₂-C₆)alkynyl, provided that both R¹ and R² are not a hydrogen atom;
R³, R⁴, and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³; or R³ and R⁴ taken together may form a fused 5, 6 or 7 membered ring which may contain up to two heteroatoms selected from the group consisting of O, S, N, and P;
R⁶, R⁷, R⁸, R⁹, R¹⁰, R¹¹, R¹², and R¹³ are each independently a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

Still yet other preferred methods of this invention use furylcarboxamides or thienylcarboxamides having the structural formula 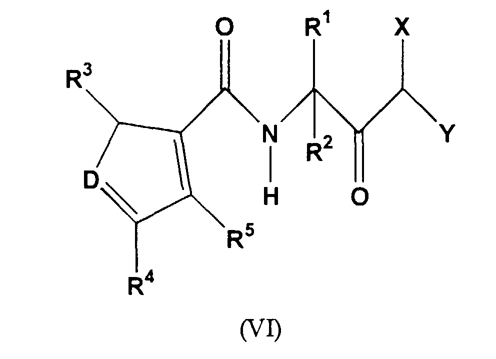 wherein
D is O or S;
R¹ and R² are each independently a hydrogen atom, (C₁-C₆)alkyl, halo(C₁-C₆)alkyl, (C₂-C₆)alkenyl, and (C₂-C₆)alkynyl, provided that both R¹ and R² are not a hydrogen atom;
R³, R⁴, and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³;
R⁶, R⁷, R⁸, R⁹, R¹⁰, R¹¹, R¹² and R¹³ are each independently a hydrogen atom or (C₁-C₆)alkyl; and
X and Y are each independently selected from a hydrogen atom, halo, cyano, thiocyano, isothiocyano and (C₁-C₆)alkylsulfonyloxy, provided that both X and Y are not a hydrogen atom; or
the optical enantiomers thereof.

When R¹ and R² are different, optical enantiomers of the compounds of the present invention are possible due to the presence of an asymmetric carbon atom linking R¹ and R². In such cases, both enantiomers are within the scope of the present invention. It is known that many biologically active compounds have optical enantiomers, one of which may bind more effectively to a particular protein than the other. Similarly, for compounds used in the method of the present invention, one enantiomer may bind more effectively to tubulin than the other enantiomer. For example, the "S enantiomers" of compounds 1, 2 and 3 bind more effectively to tubulin than the corresponding "R enantiomers". The term "S enantiomer" means that the four groups on the carbon to which R¹ and R² are attached, when ranked according to the set of sequence rules of the Cahn-Ingold-Prelog system *{Angew. Chem. Int. Ed. Engl.* **5***,* 385-415 (1966)}, define the carbon as having an S configuration. The term "R enantiomer" means that the four groups form an R configuration.

Particular compounds which have been made and which are expected to be useful in the method of the present invention include, but are not limited to, those compounds listed in Tables 1-6.

**TABLE 1:**

| **Benzoic Acid Amides of Formula (II)** | | | | | | | |
|---|---|---|---|---|---|---|---|
| Compound | R¹ | R² | R³ | R⁴ | R⁵ | X | Y |
| 1 | CH₃ | C₂H₅ | Cl | H | Cl | Cl | H |
| 2 | CH₃ | C₂H₅ | CH=NOCH₃ | NH₂ | Cl | Cl | H |
| 3 | CH₃ | C₂H₅ | Cl | CH₃ | Cl | Cl | H |
| 4 | CH₃ | C₂H₅ | H | NHCOOCH₃ | H | Cl | H |
| 5 | CH₃ | C₂H₅ | Cl | NH₂ | Cl | Cl | H |
| 6 | CH₃ | C₂H₅ | CN | H | Cl | Cl | H |
| 7 | CH₃ | C₂H₅ | CH=NOCH₃ | H | Cl | Cl | H |
| 8 | CH₃ | C₂H₅ | Br | H | CH₃ | Cl | H |
| 9 | CH₃ | C₂H₅ | Cl | H | Cl | Br | Br |
| 10 | CH₃ | CH₃ | Cl | H | Cl | Br | Cl |
| 11 | CH₃ | CH₃ | Cl | H | Cl | Br | Br |
| 12 | CH₃ | C₂H₅ | Br | NH₂ | Br | Cl | H |
| 13 | CH₃ | C₂H₅ | CN | H | H | Cl | H |
| 14 | CH₃ | C₂H₅ | Br | CH₃ | Br | Cl | H |
| 15 | CH₃ | C₂H₅ | CN | H | CH₃ | Cl | H |
| 16 | CH₃ | C₂H₅ | CH=NOCH₃ | H | H | Cl | H |
| 17 | CH₃ | C₂H₅ | Cl | H | Cl | SCN | H |
| 18 | CH₃ | CH₃ | Cl | H | Cl | NCS | H |
| 19 | CH₃ | CH₃ | Cl | H | Cl | Cl | H |
| 20 | CH₃ | CH₃ | Cl | H | Cl | Br | H |
| 21 | CH₃ | C₂H₅ | Br | CH₃ | Cl | Cl | H |
| 22 | CH₃ | C₂H₅ | Cl | F | Cl | Cl | H |
| 23 | CH₃ | C₂H₅ | Cl | Cl | Cl | Cl | H |
| 24 | CH₃ | C₂H₅ | F | H | F | Cl | H |
| 25 | CH₃ | C₂H₅ | Cl | H | H | Cl | H |
| 26 | CH₃ | C₂H₅ | F | F | F | Cl | H |
| 27 | CH₃ | isopropyl | Cl | H | Cl | Cl | H |
| 28 | CH₃ | C₂H₅ | Cl | H | Cl | F | Br |
| 29 | CH₃ | CH₃ | Cl | H | Cl | Cl | Cl |
| 30 | CH₃ | C₂H₅ | NHCOOCH₃ | H | H | Cl | H |

**TABLE 2:**

| **Pyridine Carboxamides of Formula (III)** | | | | | | |
|---|---|---|---|---|---|---|
| Compound | R¹ | R² | R³ | R⁵ | X | Y |
| 31 | CH₃ | CH₃ | Cl | Cl | Br | Br |
| 32 | CH₃ | CH₃ | Cl | Cl | Cl | H |

**TABLE 3:**

| **Pyridine Carboxamides of Formula (IV)** | | | | | | |
|---|---|---|---|---|---|---|
| Compound | R¹ | R² | R³ | R⁴ | X | Y |
| 33 | CH₃ | C₂H₅ | Br | H | Cl | H |
| 34 | CH₃ | C₂H₅ | H | Cl | Cl | H |

**TABLE 4:**

| **Thienylcarboxamides and Furylcarboxamide of Formula (V)** | | | | | | | | |
|---|---|---|---|---|---|---|---|---|
| Compound | D | R¹ | R² | R³ | R⁴ | R⁵ | X | Y |
| 35 | S | CH₃ | CH₃ | Br | Br | H | Cl | H |
| 36 | S | CH₃ | C₂H₅ | CH₃ | H | H | Cl | H |
| 37 | S | CH₃ | CH₃ | H | H | H | Br | Br |
| 38 | O | CH₃ | C₂H₅ | Br | H | H | Br | Br |

**TABLE 5:**

| **Thienylcarboxamide of Formula (VI)** | | | | | | | | |
|---|---|---|---|---|---|---|---|---|
| Compound | D | R¹ | R² | R³ | R⁴ | R⁵ | X | Y |
| 39 | S | CH₃ | CH₃ | Cl | Cl | H | Cl | H |

**TABLE 6:**

| **Fused Ring Benzoic Acid Amides of Formula (II)** | | | | | | |
|---|---|---|---|---|---|---|
| Compound | R¹ | R² | R³ | R⁴R⁵ * | X | Y |
| 40 | CH₃ | C₂H₅ | H | -N=CH-O- | Cl | H |
| 41 | CH₃ | C₂H₅ | H | -O-CH=N- | Cl | H |
| 42 | CH₃ | C₂H₅ | H | -N=CH-S- | Cl | H |
| 43 | CH₃ | C₂H₅ | Cl | -N=CH-O- | Cl | H |
| 44 | CH₃ | C₂H₅ | Cl | -N=C(CH₃)-O- | Cl | H |

| | | | | | | |
|---|---|---|---|---|---|---|
| * The left hand most atom is attached to the 4-position of the benzoic acid amide ring and the right hand most atom is attached to the 5-position of the benzoic acid amide ring. | | | | | | |

The following methods and examples further illustrate the use of the present invention.

### Methods used in preparing compounds listed in Tables 1-6

### Compounds 1, 6, 8, 9, 10, 11, 13, 15, 17, 18, 19, 20, 22, 23, 24, 25, 26, 27, 28 and 29 in Table 1:

Compounds 1, 6, 8, 9, 10, 11, 13, 15, 17, 18, 19, 20, 22, 23, 24, 25, 26, 27, 28 and 29 in Table 1 were prepared according to synthetic methods described in U.S. Patent 4,822,902, columns 5-8 and 11-17.

### Compounds 7 and 16 in Table 1:

Compounds 7 and 16 in Table 1 were prepared according to synthetic methods described in U.S. Patent 5,254,584, columns 7 and 8 (compound 16) and 10-14 (compound 7).

### Compounds 3,14 and 21 in Table 1:

Compounds 3, 14 and 21 were prepared according to synthetic methods described in U.S. Patent 5,304,572, columns 4-8.

### Compounds 5 and 12 in Table 1:

Compounds 5 and 12 were prepared using conventional synthesis techniques, as described for example in U.S. patent 4,863,940, columns 5-7, from appropriate benzoic acids or benzoyl chlorides. Thus, compounds 5 and 12 were prepared using 4-amino-3,5-dichlorobenzoylchloride and 4-amino-3,5-dibromobenzoylchloride, respectively.

### Compound 2 in Table 1:

Compound 2 was prepared by reaction of the benzoyl chloride VII, in which R³ is Cl, R⁴ is NH₂ and R⁵ is -CH=NOCH₃, with the α-amino-α'-chloroketone derivative VIII, in which R¹ is methyl and R² is ethyl, as illustrated in Scheme A:

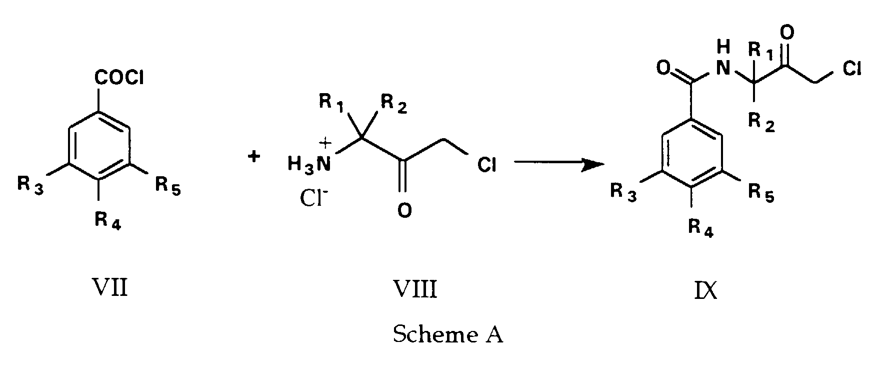

The starting benzoyl chloride used to prepare compound 2 was prepared as indicated below in scheme B.

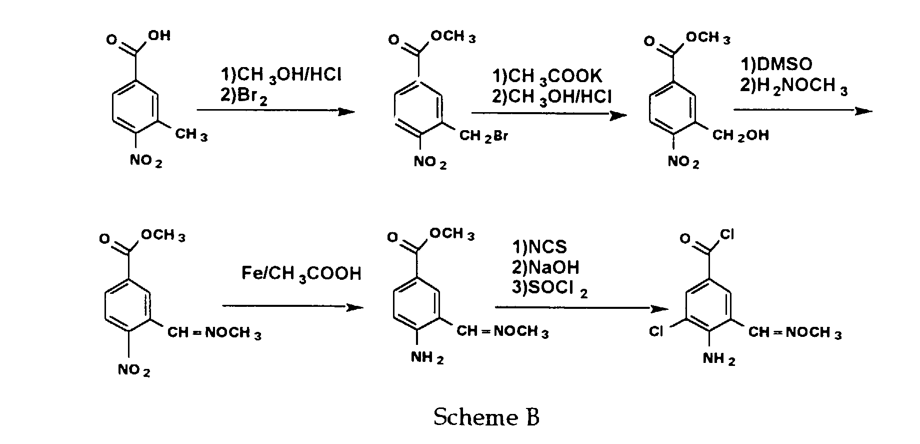

Compound VIII was prepared by treating the acetylenic amine (X) with trifluoracetic anhydride in the presence of a solvent such as methylene chloride, chloroform, ethyl ether, or water and a base such as triethylamine, sodium carbonate, sodium bicarbonate, or sodium hydroxide to yield the acetylenic amide XI: 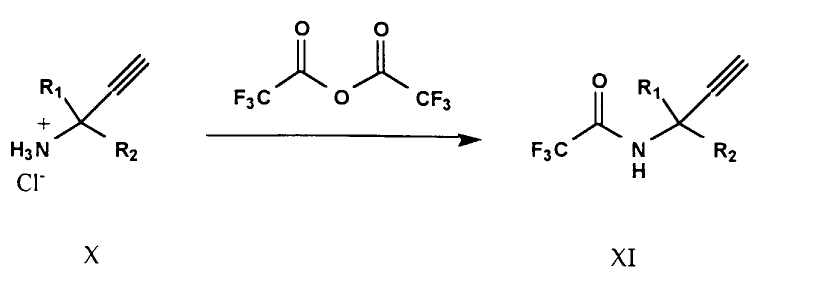 Treatment of the acetylenic amide XI with chlorine or a chlorine source at a temperature of from -78 °C to 0 °C in the presence of a solvent such as methylene chloride or chloroform yielded the intermediate oxazoline (XII). The oxazoline XII was readily hydrolyzed under acidic conditions using an acid such as hydrochloric acid or sulfuric acid with a solvent such as methanol or tetrahydrofuran at a temperature of from 40 °C to 60 °C, and yielded the α-amino-α', α'-dichloroketone (XIII). 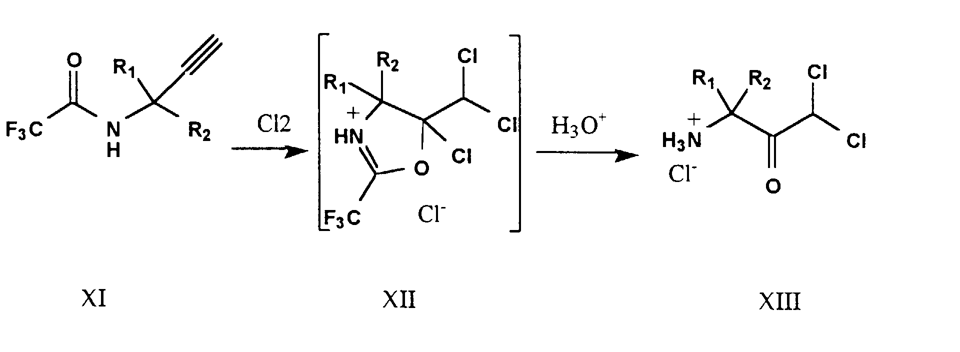 Selective catalytic dehalogenation of XIII yielded the respective α-amino-α'-chloroketone derivative VIII:

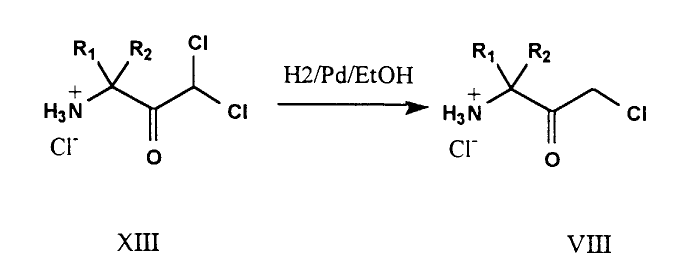

### a) Preparation of methyl 3-methyl-4-nitrobenzoate.

In a 5-liter three-necked round-bottomed flask equipped with a reflux condenser, overhead stirrer and gas inlet, was placed 300 g of 3-methyl-4-nitrobenzoic acid and 3 l of methanol. To the resulting well-stirred solution was bubbled in 20.8 g of hydrogen chloride and the resulting mixture was refluxed for 3 hours. The reaction mixture was cooled to room temperature and allowed to stand overnight. The expected methyl 3-methyl-4-nitrobenzoate precipitated as light yellow crystals, which were collected by suction filtration yielding after drying 259.3 g. This solid was used as such in the next step.

### b) Preparation of methyl 3-bromomethyl-4-nitrobenzoate

In a 5-liter three-necked round-bottomed flask equipped with a reflux condenser, overhead stirrer, addition funnel and nitrogen inlet, was placed 220 g of methyl 3-methyl-4-nitrobenzoate, 2 l of anhydrous carbon tetrachloride and 4 g of benzoyl peroxide. To the resulting solution, irradiated with a 275 watt UV light, was added 198 g of bromine dropwise over a period of 2 hours at reflux. After the addition was complete the reaction mixture was refluxed for an additional 60 hours. The reaction mixture was cooled to room temperature. The solid which formed was separated by suction filtration. This solid (159.1 g) consisted of the expected methyl 3-bromomethyl-4-nitrobenzoate with minor amounts of the starting material. The mother liquors together with another 220 g of methyl 3-methyl-4-nitrobenzoate and 4 g of benzoyl peroxide were returned to the flask and treated with 198 g of bromine as described above. After the addition was complete the reaction mixture was refluxed another 96 hours, cooled to room temperature and the resulting solid separated by filtration yielding another 252 g of methyl 3-bromomethyl-4-nitrobenzoate. The solids were combined yielding a total of 411.1 g of methyl 3-bromomethyl-4-nitrobenzoate with minor amounts of the starting methyl 3-methyl-4-nitrobenzoate and methyl 3-dibromomethyl-4-nitrobenzoate. This solid was used as such in the next step.

### c) Preparation of methyl 3-acetoxymethyl-4-nitrobenzoate.

In a 5-liter three-necked round-bottomed flask equipped with a reflux condenser, overhead stirrer and nitrogen inlet, was placed 411 g of the previously prepared methyl 3-bromomethyl-4-nitrobenzoate, 441 g of anhydrous potassium acetate and 2 l of glacial acetic acid. The resulting mixture was refluxed for 4 hours, cooled to room temperature and stirred overnight. The solvent was removed in a rotary evaporator and the resulting light yellow solid treated with a mixture of 2 l of ethyl acetate and 1 l of water. The organic phase was separated, washed with water (3x400 mL), brine (1x400 mL) dried over anhydrous magnesium sulfate and the solvent removed using a rotary evaporator. The crude reaction mixture was triturated with hexane and filtered yielding 318 g of the expected methyl 3-acetoxymethyl-4-nitrobenzoate. This compound was used as such in the next step.

### d) Preparation of methyl 3-hydroxymethyl-4-nitrobenzoate

In a 5-liter three-necked round-bottomed flask equipped with a reflux condenser, overhead stirrer and nitrogen inlet, was placed 318 g of the previously prepared methyl 3-acetoxymethyl-4-nitrobenzoate and 3.2 l of anhydrous methanol. To the resulting solution was bubbled in 40 g of hydrogen chloride and the resulting mixture was refluxed for 3 hours. After cooling to room temperature the solvent was removed using a rotary evaporator yielding 273 g of methyl 3-hydroxymethyl-4-nitrobenzoate as a yellow solid containing traces of methanol, which was used as such in the next step.

### e) Preparation of methyl 3-formyl-4-nitrobenzoate

In a 5-liter four-necked round-bottomed flask 1.5 l of methylene chloride was cooled to -78 °C. Oxalyl chloride (164 g, 1.29 moles) was added slowly, followed by dropwise addition of 202 g (2.59 moles) of dry dimethylsulfoxide in 125 mL of methylene chloride, keeping the temperature below -70 °C. After the addition was complete the reaction mixture was stirred at -78 °C for 30 minutes and 273 g (1.29 moles) of previously prepared methyl 3-hydroxymethyl-4-nitrobenzoate dissolved in 250 mL of methylene chloride was added dropwise. The reaction mixture was stirred an additional 30 minutes. Triethylamine (392 g 3.88 moles) in 125 mL of methylene chloride was added dropwise keeping the temperature below -65 °C. The reaction mixture was warmed up slowly to room temperature and stirred overnight. The solvent was removed using a rotary evaporator and the resulting solid treated with a mixture of 2 l of ethyl acetate and 1 l of water. The organic phase was separated, filtered through diatomaceous earth, and washed sequentially with dilute aqueous hydrochloric acid (2x250 mL), water (2x250 mL), saturated aqueous sodium bicarbonate (2x250 mL), water (2x200 mL), brine (1x200 mL) and dried over anhydrous magnesium sultate. The solvent was removed using a rotary evaporator. The crude reaction mixture was triturated with hexane and filtered yielding 234.1 g of the expected methyl 3-formyl-4-nitrobenzoate as a yellow solid. This compound was used as such in the next step.

### f) Preparation of methyl 3-methoxyiminomethyl-4-nitrobenzoate.

To a well stirred mixture of 195 g of methyl 3-formyl-4-nitrobenzoate, 1 l methylene chloride and 370 mL of water was added sequentially 77.6 g of methoxyamine hydrochloride, 76.2 g of sodium acetate and 6.8 g of tetra-*n*-butylammonium hydrogen sulfate. The resulting mixture was stirred overnight at room temperature, then diluted with 2 l of ethyl ether. The organic phase was separated and washed sequentially with water (1x500 mL), 2% aqueous hydrochloric acid (2x500 mL), water (2x250 mL), and brine (1x250 mL); then dried over anhydrous magnesium sulfate. The solvent was removed using a rotary evaporator yielding 218.6 g of the expected methyl 3-methoxyiminomethyl-4-nitrobenzoate as a reddish oil that solidified upon standing, and which was used as such in the next step.

### g) Preparation of methyl 4-amino-3-methoxyiminomethylbenzoate

In a 5-liter three-necked round-bottomed flask was placed 0.9 l of 5% aqueous acetic acid and 157 g (2.8 moles) of iron. To the resulting well-stirred mixture was added 166.6 g (0.7 moles) of the previously prepared methyl 3-methoxyiminomethyl-4-nitrobenzoate dissolved in 0.9 l of ethyl acetate followed by dropwise addition of 0.9 l of acetic acid while keeping the temperature below 35 °C. The resulting mixture was stirred at 35 °C for 30 minutes and filtered through diatomaceous earth. The filtrate was poured into 5 l of water. The aqueous phase was separated and washed with ethyl ether (2x500 mL). The combined organic layers were washed sequentially with water (4x500 mL), saturated aqueous sodium bicarbonate (2x500 mL), water (2x500 mL), and brine (1x400 mL). The organic layer was dried over anhydrous magnesium sulfate and the solvent removed using a rotary evaporator yielding 130 g of the expected methyl 4-amino-3-methoxyiminomethylbenzoate.

### h) Preparation of methyl 4-amino-3-chloro-5-methoxyiminomethylbenzoate.

In a 2-liter three-necked round-bottomed flask was placed 106 g (0.51 moles) of the previously prepared 4-amino-3-methoxyiminomethylbenzoate and 500 mL of acetonitrile. The resulting mixture was heated at 70 °C and 75.2 g (0.56 moles) of N-chlorosuccinimide was added portionwise while keeping the temperature below 80 °C. After the addition was complete the reaction mixture was refluxed for 1 hour. The reaction mixture was cooled to room temperature and the solvent eliminated in a rotary evaporator. The crude product was dissolved in 5 l of ethyl acetate. The organic solution was washed with water (3x500 mL) and then brine, dried over magnesium sulfate. The reaction mixture was concentrated in a rotary evaporator to a slurry, triturated with hexane and filtered yielding the expected methyl 4-amino-3-chloro-5-methoxyiminomethylbenzoate as a yellow solid. This reaction was repeated using the same amounts yielding a total of 210.5 g of methyl 4-amino-3-chloro-5-methoxyiminomethylbenzoate, which was used as such in the next step.

### i) Preparation of 4-amino-3-chloro-5-methoxyiminomethylbenzoic acid.

In a 5-liter three-necked round-bottomed flask was placed 210 g (0.86 moles) of the previously prepared 4-amino-3-chloro-5-methoxyiminomethylbenzoate, 1.7 l of methanol and 462 g (1.73 moles) of 15% aqueous sodium hydroxide. The resulting mixture was refluxed for 3 hours, after which the reaction mixture was stirred overnight at room temperature. The reaction mixture was concentrated using a rotary evaporator. The crude reaction mixture was dissolved in 2 l of water. The resulting aqueous solution was washed once with 500 mL of ethyl acetate, cooled in an ice bath and acidified to pH=2 with concentrated hydrochloric acid. The expected 4-amino-3-chloro-5-methoxyiminomethylbenzoic acid precipitated as a light yellow solid which was separated by suction filtration. The filter cake was washed with a 1:2 mixture of ethyl ether and hexane yielding after drying 185.2 g (94% yield).

### j) Preparation of 4-amino-3-chloro-5-methoxyiminomethylbenzoyl chloride.

In a 5-liter three-necked round-bottomed flask was placed 180 g of the previously prepared 4-amino-3-chloro-5-methoxyiminomethylbenzoic acid, 2 l of toluene, 3 mL of dimethylformamide and 104 g (64 mL) of thionyl chloride. The resulting mixture was heated at 70 °C for 2 hours, filtered while hot and the solvent removed using a rotary evaporator yielding 178.1 g of the expected 4-amino-3-chloro-5-methoxyiminomethylbenzoyl chloride.

### k) Preparation of 3-amino-1-chloro-3-methyl-2-pentanone hydrochloride (Compound VIII, wherein R¹ is methyl and R² is ethyl)

### (i) Preparation of N-[3-(3-methyl-1-pentynyl)]trifluoroacetamide

In a 3 liter, four-necked, round-bottomed flask fitted with a mechanical stirrer, nitrogen inlet and thermometer was placed 234 grams (g) (1.75 mole) of 3-amino-3-methyl-1-pentyne hydrochloride and 1,000 mL of methylene chloride. To the resulting well-stirred mixture was added slowly 354 g (3.51 mole) of triethylamine (TEA) dropwise, keeping the temperature below 30 °C. After the addition was completed, the reaction mixture was stirred 120 minutes followed by dropwise addition of 334.5 g (1.59 mole) of trifluoroacetic anhydride dissolved in 500 mL of methylene chloride at such a rate to keep the reaction temperature at 0 °C. After the addition was completed the reaction mixture was stirred at room temperature overnight and concentrated *in vacuo.* The resulting slurry was washed with ethyl ether. The ethyl ether layer was washed sequentially with water, saturated aqueous sodium bicarbonate and brine, dried over anhydrous magnesium sulfate, treated with activated charcoal, and filtered through Celite™. The solvent was eliminated under reduced pressure. The resulting crude product was treated with cold pentane, filtered, and dried yielding 255.5 g (83%) of the expected N-[3-(3-methyl-1-pentynyl)]trifluoroacetamide as a white solid.

### (ii) Preparation of 5-chloro-5-(dichloromethyl)-4-ethyl-4-methyl- 2-trifluoromethyloxazoline hydrochloride:

In a 5 L, four-necked, round-bottomed flask fitted with a mechanical stirrer, a thermometer, and a gas inlet was dissolved 255.5 g (1.32 mole) of N-[3-(3-methyl-1-pentynyl)]trifluoroacetamide in 4,000 mL of methylene chloride. The resulting mixture was cooled to -30 °C and 235 g of chlorine was bubbled in over a 2 hour period. When the addition was completed the reaction mixture was stirred at -30 °C during 30 minutes and warmed to room temperature. The crude reaction mixture was evaporated in the rotary evaporator yielding the expected (dichloromethyl)-4-ethyl-4-methyl-2-trifluoromethyloxazoline hydrochloride which was used as such in the next step.

### (iii) Preparation of 3-amino-1,1-dichloro-3-methyl-2-pentanone hydrochloride:

The 5-chloro-5-(dichloromethyl)-4-ethyl-4-methyl-2-trifluoromethyloxazoline hydrochloride prepared in the preceding step was dissolved in 1800 mL of methanol, 72 mL of water, and 190 mL of concentrated hydrochloric acid, warmed to 50 °C, and stirred at that temperature overnight. The crude reaction mixture was cooled and poured into an ice/water/ethyl ether mixture. The phases were separated and the ether layer was extracted once with water. The ether was saved (organic I). The combined aqueous layers were washed once with ethyl ether, and the organic layer was combined with organic I (organic II). The aqueous layer was neutralized with saturated aqueous sodium bicarbonate and extracted twice with ethyl ether. The combined ether layers were washed with water, brine, dried over anhydrous magnesium sulfate, treated with activated charcoal, and filtered through Celite™. To the resulting colorless solution was bubbled in anhydrous hydrogen chloride keeping the temperature below 20 °C. The resulting white solid was filtered and dried yielding 124.8 g of the expected 3-amino-1,1-dichloro-3-methyl-2-pentanone pentanone hydrochloride as a white solid. The ethyl ether filtrate was combined with organic II and concentrated *in vacuo ;* the resulting residue (150 g) was taken in a mixture of methanol/water/concentrated hydrochloric acid and heated at 50 °C over the weekend. The previously described workup yielded another 51 g of 3-amino-1,1-dichloro-3-methyl-2-pentanone hydrochloride. The total amount obtained was 175.8 g (61% yield).

### (iv) Preparation of 3-amino-1-chloro-3-methyl-2-pentanone hydrochloride:

In a 2 L Parr™ bottle was placed 41 g of 3-amino-1,1-dichloro-3-methyl-2-pentanone hydrochloride, 0.8 g of 10% palladium over charcoal, and 400 mL of ethanol. The resulting mixture was shaken in a Parr™ apparatus at 50 psi for 3 hours. The crude reaction mixture was filtered through Celite™ and evaporated *in vacuo* yielding a viscous oil, which was taken in 300 to 400 mL of ethyl acetate and stirred at room temperature for several hours. The expected 3-amino-1-chloro-3-methyl-2-pentanone hydrochloride crystallized as a white solid; 300 mL of hexane was added to the resulting suspension and filtered yielding 34 g (98%) of the expected 3-amino-1-chloro-3-methyl-2-pentanone hydrochloride.

The reaction was repeated starting with 41 g; 41 g; and 51 g of 3-amino-1,1-dichloro-3-methyl-2-pentanone hydrochloride yielding a total of 132.1 g (90% overall yield) of 3-amino-1-chloro-3-methyl-1-pentanone hydrochloride.

### 1) Preparation of 4-amino-3-chloro-5-methoxyiminomethyl-N-(3-chloro-1-ethyl-1-methyl-2-oxopropyl)benzamide (compound 2).

In a 5-liter three-necked round bottomed flask was placed 93 g of 3-amino-1-chloro-3-methyl-2-pentanone hydrochloride and 885 mL of water. To the resulting solution were added 138.6 g of sodium bicarbonate followed by 500 mL of ethyl acetate. To the resulting well-stirred mixture was added 123.5 g of 4-amino-3-chloro-5-methoxyiminomethylbenzoyl chloride dissolved in 1000 mL of ethyl acetate at room temperature over a period of 50 minutes. After the addition was complete the reaction mixture was stirred at room temperature for 1 hour. The two phases were separated and the organic layer was washed with water (2x500mL), brine (1x500 mL), dried over anhydrous magnesium sulfate and the solvent eliminated in a rotary evaporator yielding the crude product as a brown oil. This oil was passed through a short silica gel column using methylene chloride as elution solvent. Evaporation of the solvent yielded 133.3 g of the expected 4-amino-3-chloro-5-methoxyiminomethyl-N-(3-chloro-1-ethyl-1-methyl-2-oxopropyl)benzamide as an off-white solid (mp 140-141 °C).

### Compounds 4 and 30 in Table 1:

Compound 30 was prepared by the following procedure.

3-Nitro-N-(3-chloro-1-ethyl-1-methyl-2-oxopropyl)benzamide was prepared by reaction of 3-nitrobenzoylchloride with VIII as in scheme A (above), then converted to 3-amino-N-(3-chloro-1-ethyl-1-methyl-2-oxopropyl)benzamide by catalytic hydrogenation using palladium as the catalyst.

In a 300 ml, 4-necked, round-bottomed flask fitted with a mechanical stirrer, a thermometer and addition funnel was suspended 3.5 g of 3-amino-N-(3-chloro-1-ethyl-1-methyl-2-oxopropyl)benzamide in 100 ml of dichloromethane. The mixture was cooled to 0-5°C in an ice bath, then 1.8 ml of triethylamine was added and the mixture allowed to stir for a few minutes. Methylchloroformate (1.1 ml) was added slowly, dropwise keeping the temperature under 5°C, and the mixture stirred for 20 minutes. The ice bath was removed and the reaction mixture allowed to warm to room temperature. The reaction mixture was washed twice with 50 ml of water and dried over anhydrous magnesium sulfate. The solvent was removed using a rotary evaporator, yielding the crude product. The crude product was purified by chromatography on silica gel, yielding 230 mg of compound 30.

Compound 4 was prepared by the same procedure as compound 30, but using 4-nitrobenzoylchloride as the starting material.

### Compounds in Tables 2, 3, 4 and 5:

Compounds in Tables 2, 3, 4 and 5 were prepared according to synthetic methods described in U.S. Patent 4,863,940.

### Compounds 40, 41, 42, 43 and 44 in Table 6:

Compounds 40, 41, 43 and 44 were prepared by reaction of the corresponding aromatic derivative (VII), in which R⁴ and R⁵ together form a fused ring, with 3-amino-1-chloro-3-methyl-2-pentanone hydrochloride (compound VIII in which R¹ is methyl and R² is ethyl) as illustrated above in Scheme A:

To prepare compound 42, 6-carboxy-1,3-benzothiazole (purchased from Maybridge Chemical Company Ltd.) was first treated with thionyl chloride to give the acid chloride. The acid chloride was treated with 3-amino-1-chloro-3-methyl-2-pentanone hydrochloride in the presence of triethylamine to yield compound 42.

To prepare the aromatic portion of compound 41, the 5-carboxy benzoxazole derivative XIV was prepared from the corresponding 2-amino phenol derivative by procedures known in the art and described in, for example, E.C. Taylor, ed., The Chemistry of Heterocyclic Compounds, *vol.* 47, John Wiley & Sons, 1987 "Synthesis of Fused Heterocycles", edited by G.P. Ellis; p. 50, part I and pp. 713-714 part II). This procedure is set forth below: 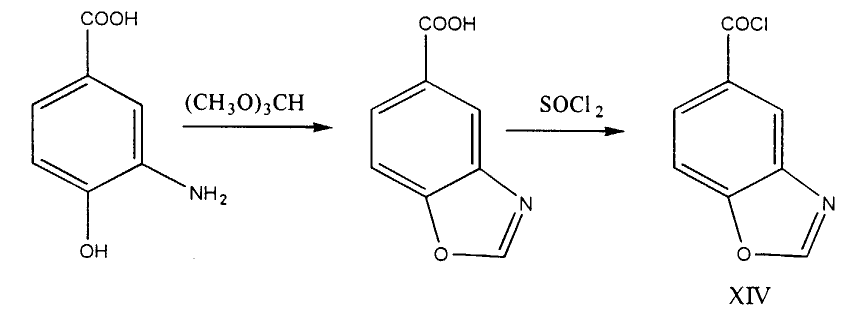 XIV was then treated with 3-amino-1-chloro-3-methyl-2-pentanone hydrochloride in the presence of triethylamine to yield compound 41.

To prepare the aromatic portions of compounds 40, 43 and 44, the 6-carboxy benzoxazole derivatives (XV) were prepared from the corresponding 2-amino phenol derivatives by the procedure set forth below: 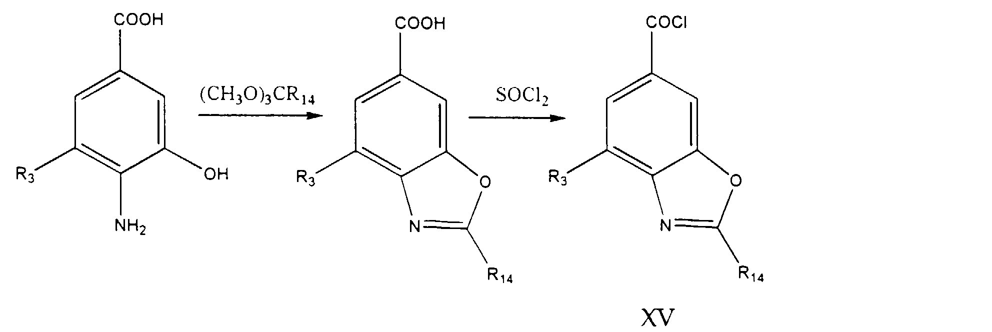 wherein R¹⁴ is H or CH₃.

To prepare compound 40, 6-carboxy-1,3-benzoxazole was prepared from 4-amino-3-hydroxy benzoic acid by treatment with trimethylorthoformate. 6-Carboxy-1,3-benzoxazole was first treated with thionyl chloride to give the acid chloride. The acid chloride was treated with 3-amino-1-chloro-3-methyl-2-pentanone hydrochloride in the presence of triethylamine to yield compound 40.

To prepare compound 43, 6-carboxy-4-chloro-1,3-benzoxazole was prepared from 4-amino-5-chloro-3-hydroxy benzoic acid by treatment with trimethylorthoformate. 6-Carboxy-4-chloro-1,3-benzoxazole was first treated with thionyl chloride to give the acid chloride. The acid chloride was treated with 3-amino-1-chloro-3-methyl-2-pentanone hydrochloride in the presence of triethylamine to yield compound 43.

To prepare compound 44, 6-carboxy-2-methyl-4-chloro-1,3-benzoxazole was prepared from 4-amino-5-chloro-3-hydroxy benzoic acid by treatment with trimethylorthoacetate. 6-Carboxy-2-methyl-4-chloro-1,3-benzoxazole was first treated with thionyl chloride to give the acid chloride. The acid chloride was treated with 3-amino-1-chloro-3-methyl-2-pentanone hydrochloride in the presence of triethylamine to yield compound 44.

The following examples are provided in order to illustrate the method of the present invention.

### Example 1: The ability of compound 3 to bind covalently to beta-tubulin when applied to the Oomycete fungus Phytophthora capsici is shown in this example.

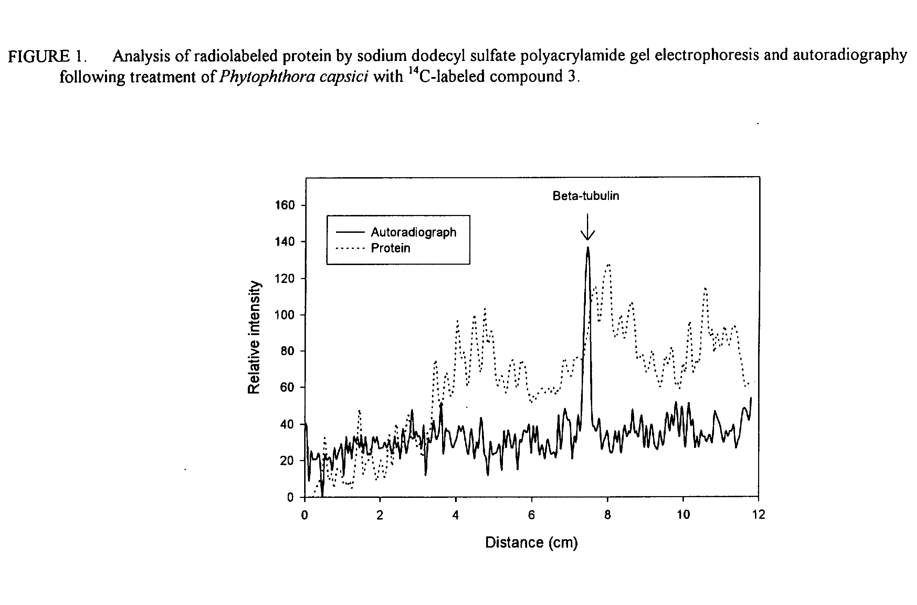

*P. capsici* (ATCC15399) was maintained on V-8 agar, pH 7.0, containing 200 ml V-8 juice, 4 g CaCO₃, and 20 g agar per liter. An erlenmeyer flask (125 ml capacity) containing 20 ml of the asparagine-sucrose medium described by Erwin, D.C. and Katznelson, K., 1971, Canadian Journal of Microbiology, vol. 7, p. 15-25 (1971) was inoculated with a mycelial plug (7 mm in diameter) cut from the growing edge of a culture of *P. capsici* grown on V-8 agar. The flask was incubated at 26°C with shaking at 120 rpm on a gyrotary shaker for 72 hours, then 50 µl of 0.8 mM ¹⁴C-labeled compound 3 was added. After further incubation for 1 hour, the fungal mycelium was collected by filtration and washed with 100 ml of water. The mycelium was frozen in liquid nitrogen and ground to a fine powder. Proteins were extracted under denaturing conditions (heating for 5 minutes at 100°C in 62 mM Tris-HCl buffer, pH 6.8, containing 3% sodium dodecyl sulfate, 5% mercaptoethanol and 10% glycerol) expected to cause dissociation of any noncovalently-bound ligand. Aliquots of the extracted proteins were subjected to sodium dodecyl sulfate polyacrylamide gel electrophoresis (SDS-PAGE) to separate the proteins according to Laemmli, U.K., Nature (London), vol. 277, pp. 680-685, 1970 on duplicate 7.5% polyacrylamide gels. One gel was stained for protein with Coomassie brilliant blue R250. Proteins from the second gel were transferred electrophoretically to a nitrocellulose membrane. The membrane was sprayed with EN³HANCE™ spray (Dupont NEN Research Products) and the location of radiolabeled protein was determined by autoradiography using Kodak X-OMAT AR film with a 3 week exposure. The location of beta-tubulin on the nitrocellulose was determined by immunolabeling with a monoclonal antibody against beta-tubulin. Results are presented in Figure 1. The dashed line represents a densitometric scan of the Coomassie stained gel and shows the distribution of proteins. The solid line represents a densitometric scan of the autoradiograph, and shows a single ¹⁴C-labeled band. The location of beta-tubulin as determined by immunolabeling is indicated by an arrow and coincided with the position of the ¹⁴C-labeled protein band.

### Example 2: The ability of compound 1 to bind covalently to beta-tubulin when applied to suspension-cultured tobacco cells is shown in this example.

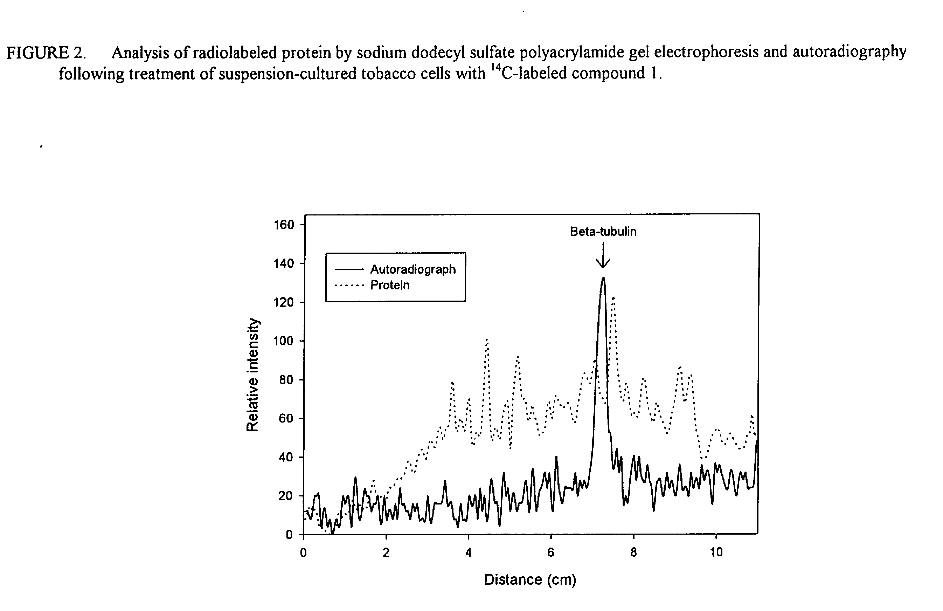

Suspension-cultured tobacco cells were grown at 27°C on a gyrotary shaker at 200 rpm in growth medium prepared according to Nagata, K. *et al.,* Molecular and General Genetics, vol. 184, pp. 161-165 (1981). Cells were treated with ¹⁴C-labeled compound 1 at a concentration of 10 µM for 1 hour, then collected by filtration. The cells were frozen in liquid nitrogen and ground to a fine powder, then the proteins were extracted and separated on duplicate gels by SDS-PAGE as described in Example 1. One gel was stained for protein and the second gel analyzed by autoradiography and immunolabeling as described in Example 1. Results are presented in Figure 2. The dashed line represents a densitometric scan of the Coomassie stained gel and shows the distribution of proteins. The solid line represents a densitometric scan of the autoradiograph, and shows a single ¹⁴C-labeled band. The location of beta-tubulin as determined by immunolabeling is indicated by an arrow and coincided with the position of the ¹⁴C-labeled protein band.

### Example 3: The ability of compound 1 to bind covalently to the beta-subunit of isolated bovine tubulin is shown in this example.

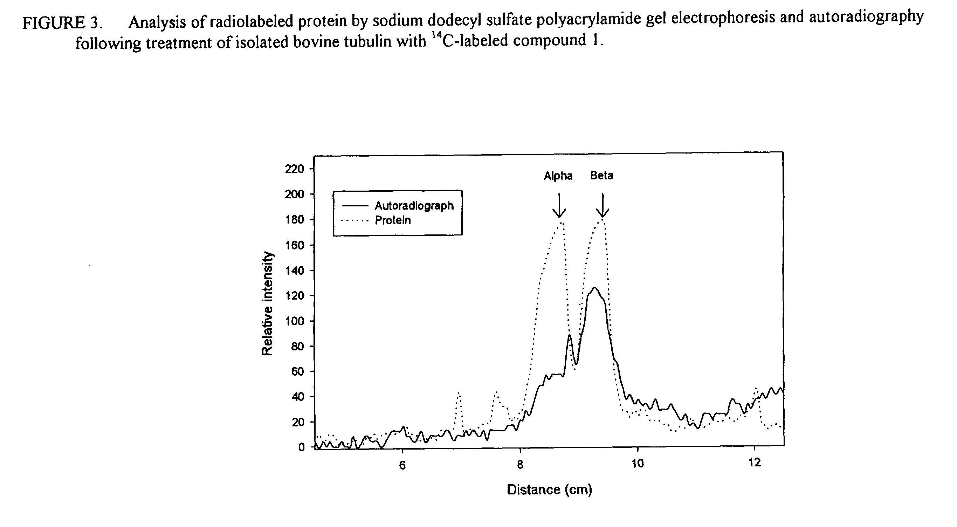

Tubulin was isolated from bovine brain using the method described by Vallee, R.B. in Methods in Enzymology vol. 134, pp. 89-104 (1986). Tubulin (10 µM) in 1.0 M sodium glutamate, pH 6.6 containing 1 mM MgCl₂ was incubated with 25 µM ¹⁴C-labeled compound 1 for 4 hours at 37°C. The sample was transferred to an ice bath for 3 minutes, and the tubulin was precipitated by addition of 20% trichloroacetic acid and incubation on ice for a further 20 minutes. After centrifugation at 12,000 g for 5 minutes the tubulin pellet was washed once with 80% ice-cold acetone, and dissolved in 62 mM Tris-HCl buffer, pH 6.8, containing 3% sodium dodecyl sulfate, 5% mercaptoethanol and 10% glycerol at room temperature. The sample was heated for 2 minutes at 100°C, then aliquots were subjected to SDS-PAGE on duplicate gels as described in Example 1. One gel was stained for protein and the second gel analyzed by autoradiography and immunolabeling as described in Example 1. Immunolabeling was carried out using monoclonal antibodies against both beta-tubulin and alpha-tubulin. Results are presented in Figure 3. The positions of alpha-tubulin and beta-tubulin as determined by immunolabelling are indicated by arrows. The dashed line represents a densitometric scan of the Coomassie stained gel and shows the distribution of proteins, with two main protein bands corresponding to alpha-tubulin and beta-tubulin. The solid line represents a densitometric scan of the autoradiograph, and shows predominant labeling of the beta-tubulin subunit.

### Example 4: The effects of various antitubulin compounds from different chemical classes on binding of compound 1 to tubulin in tobacco suspension-cultured cells is shown in this example.

Aliquots (2 ml ) of suspension-cultured tobacco cells grown as described in Example 2 were added to 25 ml capacity glass vials. Each vial then received 5 µl of DMSO (control) or 5 µl of an antitubulin compound dissolved in DMSO. Vials were incubated with shaking at 27°C for 2 hours, then 5 µl of a 100 µM solution of tritium-labeled compound 1 in DMSO was added. Vials were incubated with shaking at 27°C for 20 minutes, then binding of the radioligand was stopped by adding 5 µl of unlabeled 40 mM compound 1. The samples were incubated with shaking at 27°C for a further 20 minutes, then transferred to 15 ml capacity polypropylene centrifuge tubes in an ice bath. Each vial was rinsed with 2 ml ice-cold growth medium (described in example 2) and the rinse solution was then added to the rest of the sample. Following centrifugation at 4°C for 3 minutes at 2,500 rpm the supernatant was discarded and the pelleted cells were resuspended in 4 ml of ice-cold 10% trichloroacetic acid. Samples were kept for 1 hour in an ice bath, centrifuged again, and the cells resuspended in 4 ml of ice-cold ethanol. After incubation for a further 1 hour in an ice-bath the cells were collected by filtration on glass fiber filters and washed twice with 10 ml of ice-cold ethanol. The filters with washed cells were transferred to scintillation vials, 10 ml of Hydrofluor™ scintillation fluid (National Diagnostics, Manville, New Jersey) was added to each, and the samples were counted in a scintillation counter to determine the amount of bound radioactivity. The effects of different antitubulin compounds on binding of the radioligand are presented in Table 7 with the amounts of radioligand bound in the presence of each antitubulin compound expressed as a percentage of the amount of radioligand bound in the DMSO control. Paclitaxel and the antitubulin herbicides pronamide and chlorpropham reduced binding of the radioligand, while the antitubulin herbicide trifluralin increased binding of the radioligand.

**Table 7:**

| Effect of various antitubulin compounds on binding of tritiated compound 1 to tubulin in a whole cell binding assay using tobacco suspension-cultured cells. | | |
|---|---|---|
| **Antitubulin compound** | **Concentration (µM)** | **Binding of radioligand (% control)** |
| None (DMSO control) | | 100.0 |
| Paclitaxel | 25 | 50.3 |
| Pronamide | 100 | 68.7 |
| Trifluralin | 100 | 129.0 |
| Chlorpropham | 100 | 29.1 |

### Example 5: The effect of paclitaxel on binding of compound 1 to tubulin in Phytophthora capsici is shown in this example.

A liquid mycelial shake culture of *P. capsici* in asparagine-sucrose medium was used to prepare a homogeneous suspension of fungal material for binding assays. Asparagine-sucrose medium (100 ml) was inoculated with mycelial plugs cut from cultures grown on V-8 agar, and incubated at 26°C on a gyrotary shaker for 48 hours. The culture was homogenized in a blender, and 30 ml of the homogenate subcultured into 100 ml of fresh medium, which was then incubated at 26°C on a gyrotary shaker for 17 hours. The resulting mycelium was recovered from the medium by centrifugation and resuspended in 1170 ml of fresh medium. Aliquots (2 ml) of this suspension were added to 25 ml capacity glass vials. Each vial then received 5 µl of DMSO (control) or 5 µl of paclitaxel dissolved in DMSO. Vials were incubated with shaking at 26°C for 1 hour, then 5 µl of a 100 µM solution of tritium-labeled compound 1 in DMSO was added. Vials were incubated with shaking at 26°C for 20 min, then binding of the radioligand was stopped by adding 5 µl of unlabeled 40 mM compound 1. The samples were incubated with shaking at 27 °C for a further 20 minutes, then transferred to 15 ml capacity centrifuge tubes in an ice bath. Each vial was rinsed with 2 ml ice-cold medium which was then added to the rest of the sample. Following centrifugation at 4°C for 5 minutes at 3,400 rpm, the supernatant was discarded and the pelleted cells were resuspended in 4 ml of ice-cold 10% trichloroacetic acid. Samples were kept for 1 hour in an ice bath, centrifuged again, and the cells resuspended in 4 ml of ice-cold ethanol. After incubation for a further 2 hours in an ice-bath the cells were collected by filtration on glass fiber filters and washed twice with 10 ml of ice-cold ethanol. The filters with washed cells were transferred to scintillation vials, 10 ml of Hydrofluor™ scintillation fluid was added, and the samples were counted in a scintillation counter to determine the amount of bound radioactivity. The effect of paclitaxel at various concentrations on binding of the radioligand are presented in Table 8, with the amounts of radioligand bound in the presence of paclitaxel expressed as a percentage of the amount of radioligand bound in the DMSO control.

**Table 8:**

| Effect of paclitaxel on binding of tritiated compound 1 to tubulin in a whole cell binding assay using *Phytophthora capsici mycelium.* | | |
|---|---|---|
| **Treatment** | **Concentration (µM)** | **Binding of radioligand (% control)** |
| None (DMSO control) | | 100.0 |
| Paclitaxel | 0.004 | 84.5 |
| Paclitaxel | 0.02 | 54.6 |
| Paclitaxel | 0.1 | 34.6 |
| Paclitaxel | 0.5 | 23.6 |

### Example 6: The effect of zarilamide and compounds 7, 8, 16, 19, 23 and 27 described in Table 1 on binding of compound 1 to tubulin in Phytophthora capsici is shown in this example.

Zoospores of *P. capsici* were prepared as follows. Petri plates, 9 cm in diameter, containing 15 ml of V-8 agar were inoculated with a mycelial plug of *P. capsici* and incubated for 4 days in the dark at 25°C, then placed under continuous fluorescent light for 3 days to induce sporulation. To induce zoospore release, 15 ml of sterile water was added to each plate. The plates were incubated at room temperature for 10 minutes, followed by 20 minutes at 4°C, and finally returned to room temperature for 15 minutes. The zoospore-containing fluid was then removed from each plate and adjusted to a density of 5 × 10⁵ zoospores per ml. Assay tubes containing germinated zoospores cysts were prepared by adding 100 µl of zoospore suspension to 400 µl of asparagine-sucrose medium in 16 × 1.25 cm polypropylene tubes, which were then loosely capped and incubated on a gyrotary shaker at 120 rpm for 24 hours at 26 °C.

Tubes containing germinated zoospore cysts then received 2.5 µl of DMSO (control) or 2.5 µl of test compound dissolved in DMSO. Each compound was tested at a series of concentrations. Tubes were incubated with shaking at 26°C for 1 hour, then 2.5 µl of a 40 µM solution of tritium-labeled compound 1 in DMSO were added. Vials were incubated with shaking at 26°C for 20 minutes, then binding of the radioligand was stopped by adding 1.5 ml of ice-cold unlabeled 133 µM compound 1, and transferring the tubes to an ice bath. Following centrifugation at 3,400 rpm for 3 minutes, the supernatant was discarded and the pelleted cells were resuspended in 2 ml of ice-cold 10% trichloroacetic acid. Samples were kept for 1 hour in an ice bath, centrifuged again, and the cells resuspended in 2 ml of ice-cold ethanol. After incubation for a further 1 hour in an ice-bath, an additional 4 ml of ice-cold ethanol was added to each tube and the cells were collected by filtration on glass fiber filters in a Brandell cell harvester (Brandel Biomedical Research and Development Laboratories, Inc., Gaithersburg, Maryland), and washed twice with 4 ml of ice-cold ethanol. The filters with washed cells were transferred to scintillation vials and 0.1 ml of water was added followed by 1 ml of Protosol™ (Dupont NEN Research Products). The vials were incubated at 60°C for 30 minutes and cooled to room temperature. Ten ml of Econofluor™ scintillation fluid (Dupont NEN Research Products) was added, and the samples were counted in a scintillation counter to determine the amount of bound radioactivity. IC50 values for each test compound (defined as the concentration required to inhibit binding of the radioligand by 50% as compared to controls lacking the test compound) were determined from dose-response curves and are presented in Table 9.

**Table 9**

| Compound Compound | IC50 (µg/ml) IC50 (µg/ml) |
|---|---|
| Zarilamide | 7.02 |
| 7 | 0.98 |
| 8 | 0.25 |
| 16 | 1.17 |
| 19 | 0.25 |
| 23 | 0.06 |
| 27 | 1.83 |

### Example 7: The utility of compound 1 as a probe to detect effects of antitubulin compounds on isolated bovine tubulin is shown by this example.

Assay mixtures contained 10 µM isolated tubulin in 1.0 M sodium glutamate, pH 6.6 containing 1 mM MgCl₂, and 2.5 µl DMSO (control) or 2.5 µl of test compound dissolved in DMSO in 0.5 ml polypropylene tubes. Mixtures were incubated at 37°C for 30 minutes, then 2.5 µl of a 640 µM solution of tritium-labeled compound 1 in DMSO was added. After further incubation at 37°C for 120 minutes, aliquots (80 µl) of the assay mixtures were applied to 2.3 cm diameter Whatman 3MM filter paper disks, which were dried for 15 seconds under warm air, immersed in 3 ml of ice-cold 10% trichloroacetic acid, and left overnight at 4°C. Disks were transferred to 5% trichloroacetic acid for 30 minutes, washed twice more in 5% trichloroacetic acid, washed for 30 minutes at 37°C in ethanol/ether (1:1, v/v), then for 15 min at room temperature in ethanol/ether (1:1, v/v), and finally in two successive washes for 15 minutes each in ether at room temperature. The disks were dried, transferred to scintillation vials containing 10 ml of Hydrofluor™ and counted in a scintillation counter. The effects of antitubulin compounds at 100 µM final concentration on binding of the radioligand are presented in Table 10 with the amount of radioligand bound in the presence of each antitubulin compound expressed as a percentage of the amount of radioligand bound in the DMSO control.

**Table 10**

| Compound | Binding (% control) |
|---|---|
| Colchicine | 18.7 |
| Podophyllotoxin | 21.7 |
| Nocodazole | 31.3 |
| Vinblastine | 90.7 |

## Claims

1. Methods for carrying out binding assays, which are useful for screening compounds for antitubulin activity, resistance monitoring and determining tubulin content, in eukaryotic cells, cell extracts and tubulin preparations, using certain amides that inhibit the growth of eukaryotic cells, said amides having the structural formula 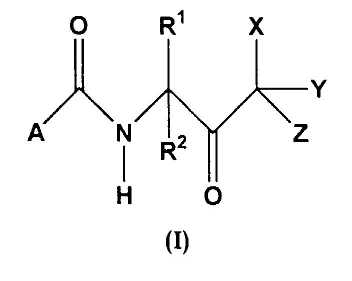 wherein
A is (C₃-C₇)cycloalkyl, (C₁-C₆)alkyl, halo(C₁-C₆)alkyl, (C₂-C₆)alkenyl, halo(C₂-C₆)alkenyl, (C₂-C₆)alkynyl, halo(C₂-C₆)alkynyl, phenyl, pyridyl, furyl, thienyl, isoxazolyl, oxazolyl, pyrrolyl, isothiazolyl, thiazolyl, pyrazolyl, imidazolyl, pyrimidinyl, quinolyl, isoquinolyl, naphthyl, pyridazinyl, pyrazinyl, benzothienyl, indolyl, benzofuranyl or benzyl; or phenyl, pyridyl, furyl, thienyl, isoxazolyl, oxazolyl, pyrrolyl, isothiazolyl, thiazolyl, pyrazolyl, imidazolyl, pyrimidinyl, quinolyl, isoquinolyl, naphthyl, pyridazinyl, pyrazinyl, benzothienyl, indolyl, benzofuranyl or benzyl substituted with four or less substituents each independently selected from halo, cyano, (C₁-C₆)alkyl, halo(C₁-C₆)alkyl, (C₂-C₆)alkenyl, halo(C₂-C₆)alkenyl, (C₂-C₆)alkynyl, halo(C₂-C₆)alkynyl, (C₁-C₆)alkoxy, halo(C₁-C₆)alkoxy, (C₁-C₆)alkylthio, halo(C₁-C₆)alkylthio, nitro, -NR⁶R⁷, -CR⁸=NOR⁹, -NHCOOR¹⁰, -CONR¹¹R¹², and -COOR¹³; or when A is an unsaturated ring and is substituted with two or more substituents which are adjacent to one another, two of said substituents may form a fused 5, 6 or 7 membered ring containing up to two heteroatoms selected from oxygen, nitrogen, sulfur and phosphorous;
R¹ and R² are each independently a hydrogen atom, (C₁-C₆)alkyl, halo(C₁-C₆)alkyl, (C₂-C₆)alkenyl, halo(C₂-C₆)alkenyl, (C₂-C₆)alkynyl or halo(C₂-C₆)alkynyl, provided that both R¹ and R² are not a hydrogen atom;
R⁶ and R⁷ are each independently a hydrogen atom, (C₁-C₆)alkyl or (C₁-C₆)alkylcarbonyl;
R⁸ is a hydrogen atom, (C₁-C₆)alkyl, (C₂-C₆)alkenyl or (C₂-C₆)alkynyl;
R⁹ is a hydrogen atom, (C₁-C₆)alkyl, (C₂-C₆)alkenyl, (C₂-C₆)alkynyl or (C₁-C₄)alkylcarbonyl;
R¹⁰, R¹¹, R¹² and R¹³ are each independently a hydrogen atom, (C₁-C₆)alkyl, (C₂-C₆)alkenyl or (C₂-C₆)alkynyl; and
X, Y and Z are each independently a hydrogen atom, halo, cyano, thiocyano, isothiocyano or (C₁-C₆)alkylsulfonyloxy, provided that at least one of X, Y and Z is not a hydrogen atom; or
the optical enantiomers thereof.

2. The methods of claim 1 using amides having the structural formula 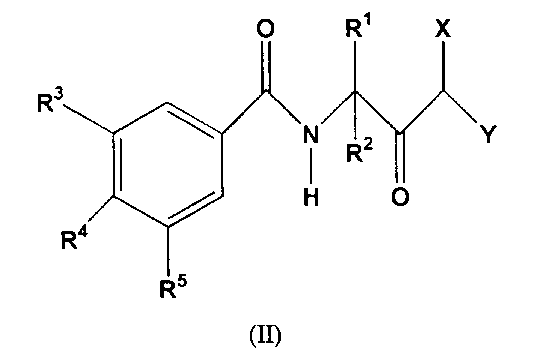 wherein
R¹ and R² are each independently a hydrogen atom, (C₁-C₆)alkyl, halo(C₁-C₆)alkyl, (C₂-C₆)alkenyl or (C₂-C₆)alkynyl, provided that both R¹ and R² are not a hydrogen atom;
R³, R⁴, and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, -NR⁶R⁷, -CR⁸=NOR⁹, -NHCOOR¹⁰, -CONR¹¹R¹² or -COOR¹³; or R⁴ and R⁵ taken together form a fused 5, 6, or 7-membered ring, which may contain up to two heteroatoms selected from the group consisting of O, S, N and P;
R⁶, R⁷, R⁸, R⁹, R¹⁰, R¹¹, R¹², and R¹³ are each independently a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

3. The methods of claim 2 using benzoic acid amides having the structural formula (II) wherein
R¹ is methyl;
R² is methyl or ethyl;
R³ and R⁵ are each independently a hydrogen atom, halo, methyl, nitro, cyano, -NR⁶R⁷, -CR⁸=NOR⁹ or -NHCOOR¹⁰, provided that both R³ and R⁵ are not a hydrogen atom;
R⁴ is a hydrogen atom, halo, -NR⁶R⁷, cyano, -CR⁸=NOR⁹, -NHCOOR¹⁰, -COOR¹³, or (C₁-C₄)alkyl;
R⁶, R⁷, R⁸, R⁹, R¹⁰ and R¹³ are each independently a hydrogen atom or (C₁-C₆)alkyl;
X is chloro; and
Y is a hydrogen atom; or
the optical enantiomers thereof.

4. In assays employing mammalian cells, cell extracts or tubulin, the methods of claim 3 using benzoic acid amides having the structural formula (II) wherein
R¹ is methyl,
R² is ethyl,
R³ is halo or cyano,
R⁴ and R⁵ are amino or -CH=NOCH₃ provided that R⁴ and R⁵ are not the same,
X is chloro, and
Y is a hydrogen atom; or
the optical enantiomers thereof.

5. In assays employing fungal cells, cell extracts or tubulin in which the fungus belongs to the Ascomycete, Deuteromycete or Basidiomycete classes of fungi, the methods of claim 3 using benzoic acid amides having the structural formula (II) wherein
R¹ is methyl,
R² is methyl or ethyl,
R³ is -CH=NOCH₃,
R⁴ is a hydrogen atom, halo, amino, cyano or methyl,
R⁵ is halo, methyl, nitro or cyano,
X is chloro and
Y is a hydrogen atom; or
the optical enantiomers thereof.

6. In assays employing fungal cells, cell extracts or tubulin in which the fungus belongs to the Oomycete class of fungi, the methods of claim 3 using benzoic acid amides having the structural formula (II) wherein
R¹ is methyl,
R² is ethyl,
R³ and R⁵ are each independently a hydrogen atom, halo, methyl, nitro, cyano, -NR⁶R⁷, -CR⁸=NOR⁹ or -NHCOOR¹⁰, provided that both R³ and R⁵ are not a hydrogen atom,
R⁴ is a hydrogen atom, halo, -NR⁶R⁷, cyano, -CR⁸=NOR⁹, -NHCOOR¹⁰, -COOR¹³ or (C₁-C₄)alkyl,
R⁶, R⁷, R⁸, R⁹, R¹⁰ and R¹³ are each independently a hydrogen atom or methyl,
X is chloro, and
Y is a hydrogen atom; or
the optical enantiomers thereof.

7. In assays employing plant cells, cell extracts or tubulin, the methods of claim 3 using benzoic acid amides having the structural formula (II) wherein
R¹ is methyl,
R² is methyl or ethyl,
R³ and R⁵ are each independently a hydrogen atom, halo, methyl, nitro or cyano, provided that both R³ and R⁵ are not a hydrogen atom,
R⁴ is a hydrogen atom,
X is chloro, and
Y is a hydrogen atom; or
the optical enantiomers thereof.

8. The methods of claim 1 using pyridinecarboxamides having the structural formula 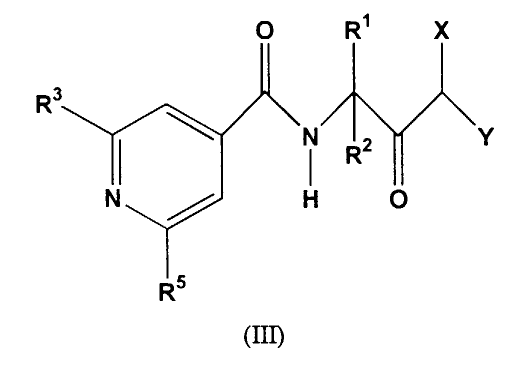 wherein
R¹ and R² are each independently a hydrogen atom, (C₁-C₆)alkyl, halo(C₁-C₆)alkyl, (C₂-C₆)alkenyl or (C₂-C₆)alkynyl, provided that both R¹ and R² are not a hydrogen atom;
R³ and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³;
R⁶, R⁷, R⁸, R⁹, R¹⁰, R¹¹, R¹², and R¹³ are a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

9. The methods of claim 1 using pyridinecarboxamides having the structural formula 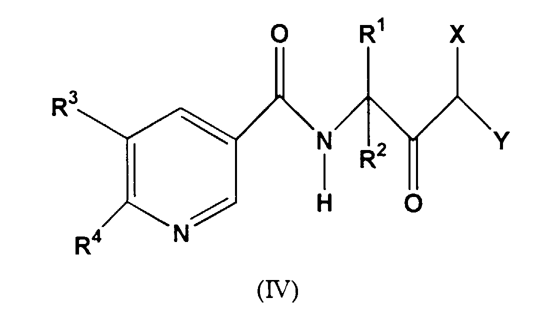 wherein
R¹ and R² are each independently a hydrogen atom, (C₁-C₆)alkyl, halo(C₁-C₆)alkyl, (C₂-C₆)alkenyl or (C₂-C₆)alkynyl, provided that both R¹ and R² are not a hydrogen atom;
R³ and R⁴ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³; or R³ and R⁴ taken together may form a fused 5, 6 or 7 membered ring which may contain up to two heteroatoms selected from the group consisting of: O, S, N, and P;
R⁶, R⁷, R⁸, R⁹, R¹⁰, R¹¹, R¹² and R¹³ are each independently a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

10. The methods of claim 9 using pyridinecarboxamides having the structural formula (IV) wherein
R¹ is methyl;
R² is methyl or ethyl;
R³ and R⁴ are each independently a hydrogen atom, halo, cyano, methyl, nitro, -NR⁶R⁷, -CR⁸=NOR⁹ or -NHCOOR¹⁰, provided that both R³ and R⁴ are not a hydrogen atom;
R⁶, R⁷, R⁸, R⁹, R¹⁰ and R¹³ are each independently a hydrogen atom or (C₁-C₆)alkyl;
X is chloro and Y is a hydrogen atom; or
the optical enantiomers thereof.

11. The methods of claim 1 using furylcarboxamides or thienylcarboxamides having the structural formula 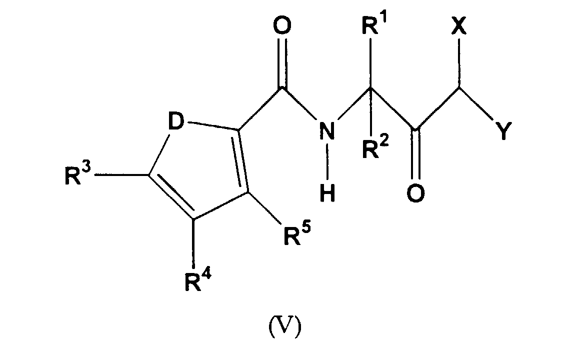 wherein
D is O or S;
R¹ and R² are each independently a hydrogen atom, (C₁-C₆)alkyl, halo(C₁-C₆)alkyl, (C₂-C₆)alkenyl, and (C₂-C₆)alkynyl, provided that both R¹ and R² are not a hydrogen atom;
R³, R⁴, and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³; or R³ and R⁴ taken together may form a fused 5, 6 or 7 membered ring which may contain up to two heteroatoms selected from the group consisting of O, S, N, and P;
R⁶, R⁷, R⁸, R⁹, R¹⁰, R¹¹, R¹², and R¹³ are each independently a hydrogen atom or (C₁-C₆)alkyl; and
X and Y are each independently a hydrogen atom, halo, cyano, thiocyano, isothiocyano or (C₁-C₆)alkylsulfonyloxy, provided that both X and Y are not a hydrogen atom; or
the optical enantiomers thereof.

12. The methods of claim 1 using furylcarboxamides or thienylcarboxamides having the structural formula 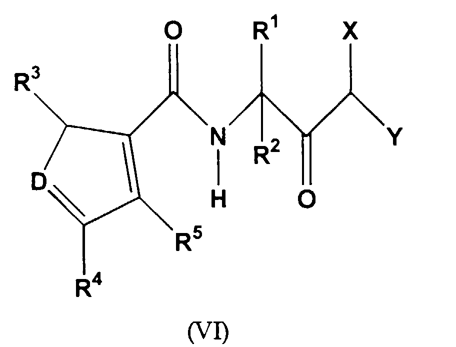 wherein
D is O or S;
R¹ and R² are each independently a hydrogen atom, (C₁-C₆)alkyl, halo(C₁-C₆)alkyl, (C₂-C₆)alkenyl, and (C₂-C₆)alkynyl, provided that both R¹ and R² are not a hydrogen atom;
R³, R⁴, and R⁵ are each independently a hydrogen atom, halo, cyano, (C₁-C₆)alkyl, halo(C₁-C₆)alkyl, (C₂-C₆)alkenyl, (C₂-C₆)alkynyl, (C₁-C₆)alkoxy, (C₁-C₆)alkylthio, halo(C₁-C₆)alkoxy, nitro, carboxy, -NR⁶R⁷, -CR⁸=NOR⁹, -NHCOOR¹⁰, -CONR¹¹R¹² or -COOR¹³;
R⁶, R⁷, R⁸, R⁹, R¹⁰, R¹¹, R¹² and R¹³ are each independently a hydrogen atom or (C₁-C₆)alkyl; and
X and Y are each independently selected from a hydrogen atom, halo, cyano, thiocyano, isothiocyano and (C₁-C₆)alkylsulfonyloxy, provided that both X and Y are not a hydrogen atom; or
the optical enantiomers thereof.

## Patentansprüche

1. Verfahren zur Durchführung von Bindungstests, die zum Screening von Verbindungen hinsichtlich der Antitubulinwirkung, Resistenzmonitoring und Bestimmen des Tubulingehalts in eukaryontischen Zellen, Zellextrakten und Tubulinpräparationen verwendbar sind, unter Verwendung von Amiden, die das Wachstum eukaryontischer Zellen inhibieren, wobei die Amide die Strukturformel 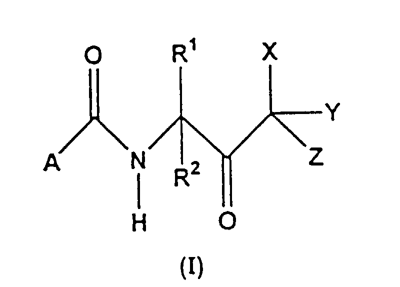 aufweisen, wobei
A (C₃-C₇)Cycloalkyl, (C₁-C₆)Alkyl, Halogen(C₁-C₆)alkyl, (C₂-C₆)Alkenyl, Halogen(C₂-C₆)alkenyl, (C₂-C₆)Alkynyl, Halogen(C₂-C₆)alkynyl, Phenyl, Pyridyl, Furyl, Thienyl, Isoxazolyl, Oxazolyl, Pyrrolyl, Isothiazolyl, Thiazolyl, Pyrazolyl, Imidazolyl, Pyrimidinyl, Chinolyl, Isochinolyl, Naphthyl, Pyridazinyl, Pyrazinyl, Benzothienyl, Indolyl, Benzofuranyl oder Benzyl, oder Phenyl, Pyridyl, Furyl, Thienyl, Isoxazolyl, Oxazolyl, Pyrrolyl, Isothiazolyl, Thiazolyl, Pyrazolyl, Imidazolyl, Pyrimidinyl, Chinolyl, Isochinolyl, Naphthyl, Pyridanzinyl, Pyrazinyl, Benzothienyl, Indolyl, Benzofuranyl oder Benzyl, substituiert mit vier oder weniger Substituenten, die jeweils unabhängig voneinander aus Halogen, Cyano, (C₁-C₆)Alkyl, Halogen(C₁-C₆)alkyl, (C₂-C₆)Alkenyl, Halogen(C₂-C₆)alkenyl, (C₂-C₆)Alkynyl, Halogen(C₂-C₆)alkynyl, (C₁-C₆)Alkoxy, Halogen(C₁-C₆)alkoxy, (C₁-C₆)Alkylthio, Halogen(C₁-C₆)alkylthio, Nitro, -NR⁶R⁷, -CR⁸ = NOR⁹, -NHCOOR¹⁰, -CONR¹¹R¹² und -COOR¹³ ausgewählt sind, oder, wenn A ein ungesättigter Ring und mit zwei oder mehr Substituenten, die zueinander benachbart sind, substituiert ist, zwei der Substituenten einen kondensierten 5-, 6- oder 7-gliedrigen Ring bilden, der bis zu zwei Heteroatome, die aus Sauerstoff, Stickstoff, Schwefel und Phosphor ausgewählt sind, enthält, bedeutet,
R¹ und R² unabhängig voneinander ein Wasserstoffatom, (C₁-C₆)Alkyl, Halogen(C₁-C₆)alkyl, (C₂-C₆)Alkenyl, Halogen(C₂-C₆)alkenyl, (C₂-C₆)Alkynyl oder Halogen(C₂-C₆)alkynyl bedeuten, mit der Maßgabe, daß sowohl R¹ als auch R² kein Wasserstoffatom bedeuten,
R⁶ und R⁷ unabhängig voneinander ein Wasserstoffatom, (C₁-C₆)Alkyl oder (C₁-C₆)Alkylcarbonyl bedeuten,
R⁸ ein Wasserstoffatom, (C₁-C₆)Alkyl, (C₂-C₆)Alkenyl oder (C₂-C₆)Alkynyl bedeutet,
R⁹ ein Wasserstoffatom, (C₁-C₆)Alkyl, (C₂-C₆)Alkenyl, (C₂-C₆)Alkynyl oder (C₁-C₄)Alkylcarbonyl bedeutet,
R¹⁰, R¹¹, R¹² und R¹³ jeweils unabhängig voneinander ein Wasserstoffatom, (C₁-C₆)Alkyl, (C₂-C₆)Alkenyl oder (C₂-C₆)Alkynyl bedeuten und
X, Y und Z jeweils unabhängig voneinander ein Wasserstoffatom, Halogen, Cyano, Thiocyano, Isothiocyano oder (C₁-C₆)Alkylsulfonyloxy bedeuten, mit der Maßgabe, daß eines von X, Y und Z kein Wasserstoffatom bedeutet, oder
den optischen Enantiomeren davon.

2. Verfahren nach Anspruch 1 unter Verwendung von Amiden mit der Strukturformel 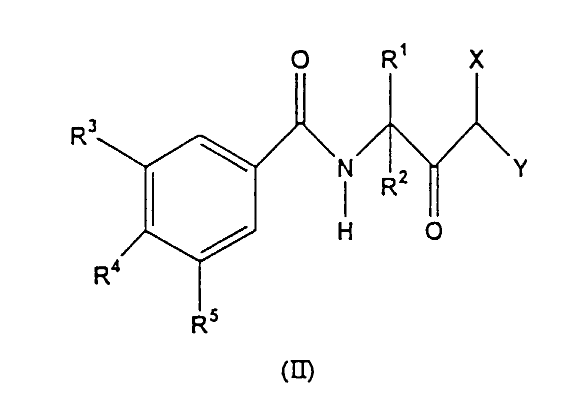 wobei
R¹ und R² unabhängig voneinander ein Wasserstoffatom, (C₁-C₆)Alkyl, Halogen(C₁-C₆)Alkyl, (C₂-C₆)Alkenyl oder (C₂-C₆)Alkynyl bedeuten, mit der Maßgabe, daß sowohl R¹ als R² kein Wasserstoffatom bedeuten,
R³, R⁴ und R⁵ jeweils unabhängig voneinander ein Wasserstoffatom, Halogen, Cyano, (C₁-C₆)Alkyl, Halogen(C₁-C₆)alkyl, (C₂-C₆)Alkenyl, (C₂-C₆)Alkynyl, (C₁-C₆)Alkoxy, (C₁-C₆)Alkylthio, Halogen(C₁-C₆)alkoxy, Nitro, -NR⁶R⁷, -CR⁸=NOR⁹, -NHCOOR¹⁰, -CONR¹¹R¹² oder -COOR¹³ bedeuten, oder R⁴ und R⁵ zusammen einen kondensierten 5-, 6- oder 7-gliedrigen Ring bilden, der bis zu zwei Heteroatome, die aus der Gruppe, bestehend aus O, S, N und P, ausgewählt sind, enthalten kann,
R⁶, R⁷, R⁸, R⁹, R¹⁰, R¹¹, R¹² und R¹³ jeweils unabhängig voneinander ein Wasserstoffatom oder (C₁-C₆)Alkyl bedeuten und
X und Y unabhängig voneinander ein Wasserstoffatom, Halogen, Cyano, Thiocyano, Isothiocyano oder (C₁-C₆)Alkylsulfonyloxy bedeuten, mit der Maßgabe, daß sowohl X als auch Y kein Wasserstoffatom bedeuten, oder den optischen Enantiomeren davon.

3. Verfahren nach Anspruch 2 unter Verwendung von Benzoesäureamiden mit der Strukturformel (II), wobei
R¹ Methyl bedeutet,
R² Methyl oder Ethyl bedeutet,
R³ und R⁵ jeweils unabhängig voneinander ein Wasserstoffatom, Halogen, Methyl, Nitro, Cyano, -NR⁶R⁷, -CR⁸ = NOR⁹ oder -NHCOOR¹⁰ bedeuten, mit der Maßgabe, daß R³ und R⁵ kein Wasserstoffatom bedeuten,
R⁴ ein Wasserstoffatom, Halogen, -NR⁶R⁷, Cyano, -CR⁸ = NOR⁹,
-NHCOOR¹⁰, -COOR¹³ oder (C₁-C₄)Alkyl bedeutet,
R⁶, R⁷, R⁸, R⁹, R¹⁰ und R¹³ jeweils unabhängig voneinander ein Wasserstoff oder (C₁-C₆)Alkyl bedeuten,
X Chlor bedeutet und
Y ein Wasserstoffatom bedeutet, oder
den optischen Enantiomeren davon.

4. Verfahren nach Anspruch 3 in Tests, welche Säugerzellen, Zellextrakte oder Tubulin verwenden, unter Verwendung von Benzoesäureamiden mit der Strukturformel (II), wobei
R¹ Methyl bedeutet,
R² Ethyl bedeutet,
R³ Halogen oder Cyano bedeutet,
R⁴ und R⁵ Amino oder -CH = NOCH₃ bedeuten, mit der Maßgabe, daß R⁴ und R⁵ nicht gleich sind,
X Chlor bedeutet und
Y ein Wasserstoffatom bedeutet, oder
den optischen Enantiomeren davon.

5. Verfahren nach Anspruch 3 in Tests, die pilzartige Zellen, Zellextrakte oder Tubulin verwenden, wobei der Pilz den Ascomycet-, Deuteromycet-oder Basidiomycetklassen der Pilze angehört, unter Verwendung von Benzoesäureamiden mit Strukturformel (II), wobei
R¹ Methyl bedeutet,
R² Methyl oder Ethyl bedeutet,
R³ -CH = NOCH₃ bedeutet,
R⁴ ein Wasserstoffatom, Halogen, Amino, Cyano oder Methyl bedeutet,
R⁵ Halogen, Methyl, Nitro oder Cyano bedeutet,
X Chlor bedeutet und
Y ein Wasserstoffatom bedeutet, oder
den optischen Enantiomeren davon.

6. Verfahren nach Anspruch 3 in Tests, die pilzartige Zellen, Zellextrakte oder Tubulin verwenden, in welchen der Pilz der Oomycetklasse der Pilze angehört, unter Verwendung von Benzoesäureamiden mit der Strukturformel (II), wobei
R¹ Methyl bedeutet,
R² Ethyl bedeutet,
R³ und R⁵ jeweils unabhängig voneinander ein Wasserstoffatom, Halogen, Methyl, Nitro, Cyano, -NR⁶R⁷, -CR⁸=NOR⁹ oder -NHCOOR¹⁰ bedeuten, mit der Maßgabe, daß sowohl R³ als auch R⁵ kein Wasserstoff bedeutet, R⁴ ein Wasserstoffatom, Halogen, -NR⁶R⁷, Cyano, -CR⁸ = NOR⁹,
-NHCOOR¹⁰, -COOR¹³ oder (C₁-C₄)Alkyl bedeutet,
R⁶, R⁷, R⁸, R⁹, R¹⁰ und R¹³ jeweils unabhängig voneinander ein Wasserstoffatom oder Methyl bedeuten,
X Chlor bedeutet und
Y ein Wasserstoffatom bedeutet, oder
den optischen Enantiomeren davon.

7. Verfahren nach Anspruch 3 in Tests, die Pflanzenzellen, Zellextrakte oder Tubulin verwenden, unter Verwendung von Benzoesäureamiden mit der Strukturformel (II), wobei
R¹ Methyl bedeutet,
R² Methyl oder Ethyl bedeutet,
R³ und R⁵ jeweils unabhängig voneinander ein Wasserstoffatom, Halogen, Methyl, Nitro oder Cyano bedeuten, mit der Maßgabe, daß sowohl R³ als auch R⁵ kein Wasserstoffatom bedeuten,
R⁴ ein Wasserstoffatom bedeutet,
X Chlor bedeutet und
Y ein Wasserstoffatom bedeutet, oder
den optischen Enantiomeren davon.

8. Verfahren nach Anspruch 1 unter Verwendung von Pyridincarboxamiden mit der Strukturformel 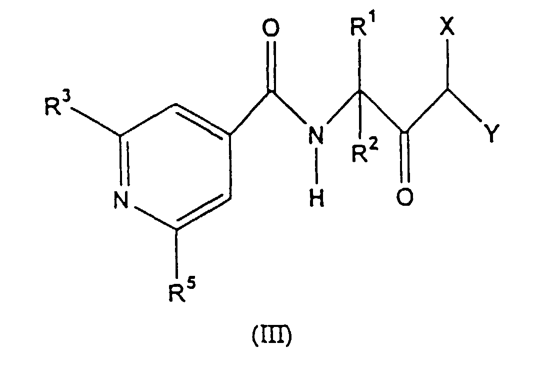 wobei
R¹ und R² jeweils unabhängig voneinander ein Wasserstoffatom, (C₁-C₆)Alkyl, Halogen(C₁-C₆)alkyl, (C₂-C₆)Alkenyl oder (C₂-C₆)Alkynyl bedeuten, mit der Maßgabe, daß sowohl R¹ als auch R² kein Wasserstoffatom bedeuten,
R³ und R⁵ jeweils unabhängig voneinander ein Wasserstoffatom, Halogen, Cyano, (C₁-C₆)Alkyl, Halogen(C₁-C₆)alkyl, (C₂-C₆)Alkenyl, (C₂-C₆)Alkynyl, (C₁-C₆)Alkoxy, (C₁-C₆)Alkylthio, Halogen(C₁-C₆)alkoxy, Nitro, Carboxy, -NR⁶R⁷, -CR⁸=NOR⁹, -NHCOOR¹⁰, -CONR¹¹R¹² oder -COOR¹³ bedeuten, R⁶, R⁷, R⁸, R⁹, R¹⁰, R¹¹, R¹² und R¹³ ein Wasserstoffatom oder (C₁-C₆)Alkyl bedeuten und X und Y jeweils unabhängig voneinander ein Wasserstoffatom, Halogen, Cyano, Thiocyano, Isothiocyano oder (C₁-C₆)Alkylsulfonyloxy bedeuten, mit der Maßgabe, daß sowohl X als auch Y kein Wasserstoffatom bedeuten, oder
den optischen Enantiomeren davon.

9. Verfahren nach Anspruch 1 unter Verwendung von Pyridincarboxamiden mit der Strukturformel 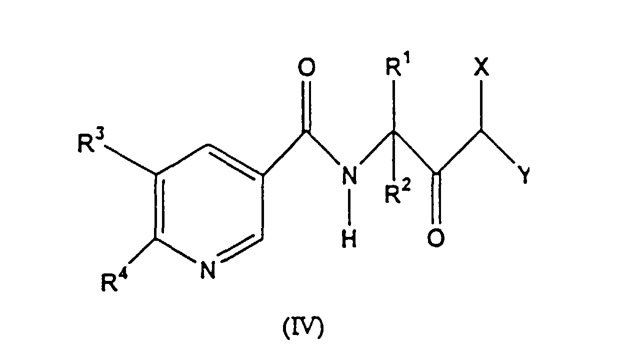 wobei
R¹ und R² jeweils unabhängig voneinander ein Wasserstoffatom, (C₁-C₆)Alkyl, Halogen(C₁-C₆)alkyl, (C₂-C₆)Alkenyl oder (C₂-C₆)Alkynyl bedeuten, mit der Maßgabe, daß sowohl R¹ als auch R² kein Wasserstoffatom bedeuten,
R³ und R⁴ jeweils unabhängig voneinander ein Wasserstoffatom, Halogen, Cyano, (C₁-C₆)Alkyl, Halogen(C₁-C₆)alkyl, (C₂-C₆)Alkenyl, (C₂-C₆)Alkynyl, (C₁-C₆)Alkoxy, (C₁-C₆)Alkylthio, Halogen(C₁-C₆)alkoxy, Nitro, Carboxy, -NR⁶R⁷, -CR⁸=NOR⁹, -NHCOOR¹⁰, -CONR¹¹R¹² oder -COOR¹³ bedeuten oder R³ und R⁴ zusammen einen kondensierten 5-, 6- oder 7-gliedrigen Ring bilden können, der bis zu zwei Heteroatome enthalten kann, die aus der Gruppe, bestehend aus O, S, N und P ausgewählt sind,
R⁶, R⁷, R⁸, R⁹, R¹⁰, R¹¹, R¹² und R¹³ jeweils unabhängig voneinander ein Wasserstoffatom oder (C₁-C₆)Alkyl bedeuten und
X und Y jeweils unabhängig voneinander ein Wasserstoffatom, Halogen, Cyano, Thiocyano, Isothiocyano oder (C₁-C₆)Alkylsulfonyloxy bedeuten, mit der Maßgabe, daß sowohl X als auch Y kein Wasserstoffatom bedeuten, oder den optischen Enantiomeren davon.

10. Verfahren nach Anspruch 9 unter Verwendung von Pyridincarboxamiden mit der Strukturformel (IV), wobei
R¹ Methyl bedeutet,
R² Methyl oder Ethyl bedeutet,
R³ und R⁴ jeweils unabhängig voneinander ein Wasserstoffatom, Halogen, Cyano, Methyl, Nitro, -NR⁶R⁷, -CR⁸=NOR⁹ oder -NHCOOR¹⁰ bedeuten, mit der Maßgabe, daß sowohl R³ als auch R⁴ kein Wasserstoffatom bedeuten,
R⁶, R⁷, R⁸, R⁹, R¹⁰ und R¹³ jeweils unabhängig voneinander ein Wasserstoffatom oder (C₁-C₆)Alkyl bedeuten,
X Chlor bedeutet und Y ein Wasserstoffatom bedeutet, oder
den optischen Enantiomeren davon.

11. Verfahren nach Anspruch 1 unter Verwendung von Furylcarboxamiden oder Thienylcarboxamiden mit der Strukturformel 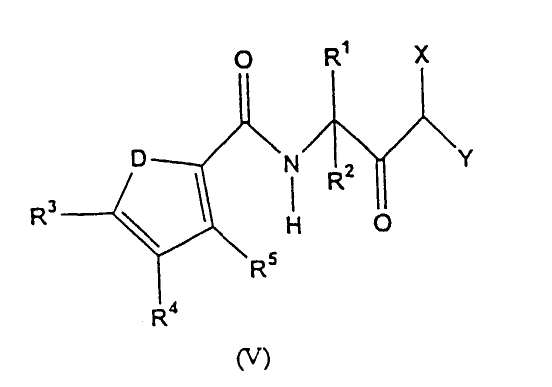 wobei
D O oder S bedeutet,
R¹ und R² jeweils unabhängig voneinander ein Wasserstoffatom, (C₁-C₆)Alkyl, Halogen(C₁-C₆)alkyl, (C₂-C₆)Alkenyl und (C₂-C₆)Alkynyl bedeuten, mit der Maßgabe, daß sowohl R¹ als auch R² kein Wasserstoffatom bedeuten, R³, R⁴ und R⁵ jeweils unabhängig voneinander ein Wasserstoffatom, Halogen, Cyano, (C₁-C₆)Alkyl, Halogen(C₁-C₆)Alkyl, (C₂-C₆)Alkenyl, (C₂-C₆)Alkynyl, (C₁-C₆)Alkoxy, (C₁-C₆)Alkylthio, Halogen(C₁-C₆)alkoxy, Nitro, Carboxy, -NR⁶R⁷, -CR⁸=NOR⁹, -NHCOOR¹⁰, -CONR¹¹R¹² oder -COOR¹³ bedeuten, oder R³ oder R⁴ zusammen einen kondensierten 5-, 6-oder 7-gliedrigen Ring bilden können, der bis zu zwei Heteroatome enthalten kann, die aus der Gruppe, bestehend aus O, S, N und P, ausgewählt sind,
R⁶, R⁷, R⁸, R⁹, R¹⁰, R¹¹, R¹² und R¹³ jeweils unabhängig voneinander ein Wasserstoffatom oder (C₁-C₆)Alkyl bedeuten und
X und Y jeweils unabhängig voneinander ein Wasserstoffatom, Halogen, Cyano, Thiocyano, Isothiocyano oder (C₁-C₆)Alkylsulfonyloxy bedeuten, mit der Maßgabe, daß sowohl X als auch Y kein Wasserstoffatom bedeuten, oder den optischen Enantiomeren davon.

12. Verfahren nach Anspruch 1 unter Verwendung von Furylcarboxamiden oder Thienylcarboxamiden mit der Strukturformel 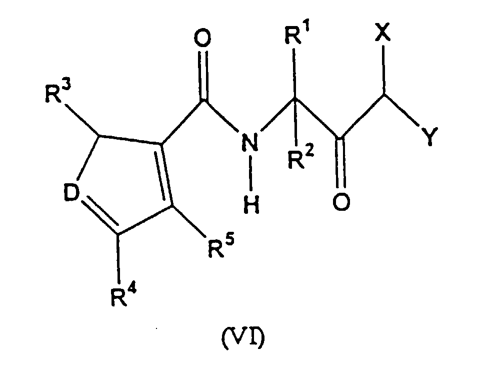 wobei
D O oder S bedeutet,
R¹ und R² jeweils unabhängig voneinander ein Wasserstoffatom, (C₁-C₆)Alkyl, Halogen(C₁-C₆)alkyl, (C₂-C₆)Alkenyl und (C₂-C₆)Alkynyl bedeuten, mit der Maßgabe, daß sowohl R¹ und R² kein Wasserstoffatom bedeuten,
R³, R⁴ und R⁵ jeweils unabhängig voneinander ein Wasserstoffatom, Halogen, Cyano, (C₁-C₆)Alkyl, Halogen(C₁-C₆)alkyl, (C₂-C₆)Alkenyl, (C₂-C₆)Alkynyl, (C₁-C₆)Alkoxy, (C₁-C₆)Alkylthio, Halogen(C₁-C₆)alkoxy, Nitro, Carboxy, -NR⁶R⁷, -CR⁸=NOR⁹, -NHCOOR¹⁰, -CONR¹¹R¹² oder -COOR¹³ bedeuten,
R⁶, R⁷, R⁸, R⁹, R¹⁰, R¹¹, R¹² und R¹³ jeweils unabhängig voneinander ein Wasserstoffatom oder (C₁-C₆)Alkyl bedeuten, und
X und Y jeweils unabhängig voneinander aus einem Wasserstoffatom, Halogen, Cyano, Thiocyano, Isothiocyano und (C₁-C₆)Alkylsulfonyloxy ausgewählt sind, mit der Maßgabe, daß sowohl X als auch Y kein Wasserstoffatom bedeuten, oder
den optischen Enantiomeren davon.

## Revendications

1. Procédés pour la mise en oeuvre de tests de fixation, qui sont utiles pour sélectionner des composés pour leur activité antitubuline, pour contrôler la résistance et pour déterminer la teneur en tubuline, dans des cellules eucaryotes, des extraits cellulaires et des préparations de tubuline, en utilisant certains amides qui inhibent la croissance des cellules eucaryotes, lesdits amides ayant la formule développée suivante: 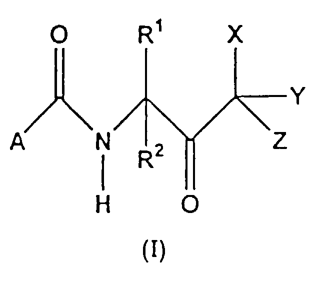 dans laquelle
A est un groupe cycloalkyle en C₃-C₇, alkyle en C₁-C₆, halogénoalkyle en C₁-C₆, alcényle en C₂-C₆, halogénoalcényle en C₂-C₆, alcynyle en C₂-C₆, halogénoalcynyle en C₂-C₆, phényle, pyridyle, furyle, thiényle, isoxazolyle, oxazolyle, pyrrolyle, isothiazolyle, thiazolyle, pyrazolyle, imidazolyle, pyrimidinyle, quinoléyle, isoquinoléyle, naphtyle, pyridazinyle, pyrazinyle, benzothiényle, indolyle, benzofuranyle ou benzyle; ou un groupe phényle, pyridyle, furyle, thiényle, isoxazolyle, oxazolyle, pyrrolyle, isothiazolyle, thiazolyle, pyrazolyle, imidazolyle, pyrimidinyle, quinoléyle, isoquinoléyle, naphtyle, pyridazinyle, pyrazinyle, benzothiényle, indolyle, benzofuranyle ou benzyle, substitué par quatre substituants ou moins, chacun étant choisi indépendamment parmi les halogènes, les groupes cyano, alkyle en C₁-C₆, halogénoalkyle en C₁-C₆, alcényle en C₂-C₆, halogénoalcényle en C₂-C₆, alcynyle en C₂-C₆, halogénoalcynyle en C₂-C₆, alcoxy en C₁-C₆, halogénoalcoxy en C₁-C₆, alkylthio en C₁-C₆, halogénoalkylthio en C₁-C₆, nitro, -NR⁶R⁷, -CR⁸=NOR⁹, -NHCOOR¹⁰, -CONR¹¹R¹² et -COOR¹³; ou lorsque A est un noyau insaturé et est substitué par deux ou plusieurs substituants qui sont en position adjacente les uns par rapport aux autres, deux desdits substituants peuvent former un noyau condensé à 5, 6 ou 7 chaînons, contenant jusqu'à deux hétéroatomes choisis parmi les atomes d'oxygène, d'azote, de soufre et de phosphore;
R¹ et R² représentent chacun indépendamment un atome d'hydrogène, un groupe alkyle en C₁-C₆, halogénoalkyle en C₁-C₆, alcényle en C₂-C₆, halogénoalcényle en C₂-C₆, alcynyle en C₂-C₆ ou halogénoalcynyle en C₂-C₆, pourvu que R¹ et R² ne soient pas tous deux des atomes d'hydrogène;
R⁶ et R⁷ représentent chacun indépendamment un atome d'hydrogène, un groupe alkyle en C₁-C₆ ou alkyl(en C₁-C₆) carbonyle;
R⁸ est un atome d'hydrogène, un groupe alkyle en C₁-C₆, alcényle en C₂-C₆ ou alcynyle en C₂-C₆;
R⁹ est un atome d'hydrogène, un groupe alkyle en C₁-C₆, alcényle en C₂-C₆, alcynyle en C₂-C₆ ou alkyl(en C₁-C₄)carbonyle;
R¹⁰, R¹¹, R¹² et R¹³ représentent chacun indépendamment un atome d'hydrogène, un groupe alkyle en C₁-C₆, alcényle en C₂-C₆ ou alcynyle en C₂-C₆; et
X, Y et Z représentent chacun indépendamment un atome d'hydrogène, un halogéno, un groupe cyano, thiocyano, isothiocyano ou alkyl(en C₁-C₆)sulfonyloxy, pourvu qu'au moins un des radicaux X, Y et Z ne soit pas un atome d'hydrogène; ou
leurs énantiomères optiques.

2. Procédés selon la revendication 1 utilisant des amides ayant la formule développée suivante: 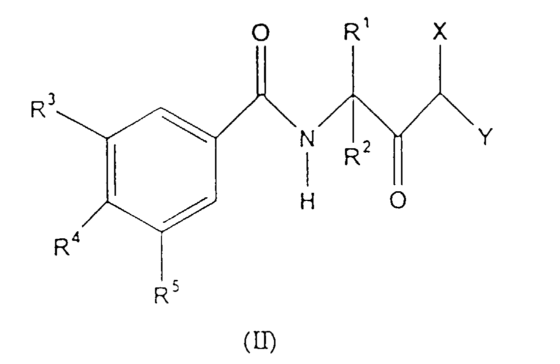 dans laquelle
R¹ et R² représentent chacun indépendamment un atome d'hydrogène, un groupe alkyle en C₁-C₆, halogénoalkyle en C₁-C₆, alcényle en C₂-C₆, ou alcynyle en C₂-C₆, pourvu que R¹ et R² ne pas tous deux des atomes d'hydrogène;
R³, R⁴, et R⁵ représentent chacun indépendamment un atome d'hydrogène, un halogéno, un groupe cyano, alkyle en C₁-C₆, halogénoalkyle en C₁-C₆, alcényle en C₂-C₆, alcynyle en C₂-C₆, alcoxy en C₁-C₆, alkylthio en C₁-C₆, halogénoalcoxy en C₁-C₆, nitro, -NR⁶R⁷, -CR⁸=NOR⁹, -NHCOOR¹⁰, -CONR¹¹R¹² ou -COOR¹³; ou R⁴ et R⁵ forment ensemble un noyau condensé à 5, 6 ou 7 chaînons, qui peut contenir jusqu'à deux hétéroatomes choisis dans le groupe constitué par O, S, N et P;
R⁶, R⁷, R⁸, R⁹, R¹⁰, R¹¹, R¹² et R¹³ représentent chacun indépendamment un atome d'hydrogène ou un groupe alkyle en C₁-C₆; et
X et Y représentent chacun indépendamment un atome d'hydrogène, un halogéno, un groupe cyano, thiocyano, isothiocyano ou alkyl(en C₁-C₆)sulfonyloxy, pourvu que X et Y ne soient pas tous deux des atomes d'hydrogène; ou
leurs énantiomères optiques.

3. Procédés selon la revendication 2 utilisant des amides d'acide benzoïque ayant la formule développée (II) dans laquelle
R¹ est un groupe méthyle;
R² est un groupe méthyle ou éthyle;
R³ et R⁵ représentent chacun indépendamment un atome d'hydrogène, un halogéno, un groupe méthyle, nitro, cyano, -NR⁶R⁷, -CR⁸=NOR⁹ ou -NHCOOR¹⁰, pourvu que R³ et R⁵ ne soient pas tous deux des atomes d'hydrogène;
R⁴ est un atome d'hydrogène, un halogéno, un groupe -NR⁶R⁷, cyano, -CR⁸=NOR⁹, -NHCOOR¹⁰, -COOR¹³ ou allyle en C₁-C₄;
R⁶, R⁷, R⁸, R⁹, R¹⁰ et R¹³ représentent chacun indépendamment un atome d'hydrogène ou un groupe alkyle en C₁-C₆;
X est un chloro; et
Y est un atome d'hydrogène; ou
leurs énantiomères optiques.

4. Dans des tests employant des cellules de mammifères, des extraits cellulaires ou de la tubuline, procédés selon la revendication 3 utilisant des amides d'acide benzoïque ayant la formule développée (II) dans laquelle
R¹ est un groupe méthyle,
R² est un groupe éthyle,
R³ est un halogéno ou un cyano,
R⁴ et R⁵ sont des groupes amino ou -CH=NOCH₃, pourvu que R⁴ et R⁵ ne soient pas identiques,
X est un chloro, et
Y est un atome d'hydrogène; ou
leurs énantiomères optiques.

5. Dans des tests employant des cellules fongiques, des extraits cellulaires ou de la tubuline, dans lesquels le champignon appartient aux classes de champignons ascomycètes, deutéromycètes ou basidiomycètes, procédés selon la revendication 3 utilisant des amides d'acide benzoïque ayant la formule développée (II) dans laquelle
R¹ est un groupe méthyle,
R² est un groupe méthyle ou éthyle,
R³ est -CH=NOCH₃,
R⁴ est un atome d'hydrogène, un halogéno, un groupe amino, cyano ou méthyle,
R⁵ est un halogéno, un groupe méthyle, nitro ou cyano,
X est un chloro et
Y est un atome d'hydrogène; ou
leurs énantiomères optiques.

6. Dans des tests employant des cellules fongiques, des extraits cellulaires ou de la tubuline, dans lesquels le champignon appartient à la classe de champignons oomycètes, procédés selon la revendication 3 utilisant des amides d'acide benzoïque ayant la formule développée (II) dans laquelle
R¹ est un groupe méthyle,
R² est un groupe éthyle,
R³ et R⁵ représentent chacun indépendamment un atome d'hydrogène, un halogéno, un groupe méthyle, nitro, cyano, -NR⁶R⁷, -CR⁸=NOR⁹ ou -NHCOOR¹⁰, pourvu que R³ et R⁵ ne soient pas tous deux des atomes d'hydrogène;
R⁴ est un atome d'hydrogène, un halogéno, un groupe -NR⁶R⁷, cyano, -CR⁸=NOR⁹, -NHCOOR¹⁰, -COOR¹³ ou alkyle en C₁-C₄;
R⁶, R⁷, R⁸, R⁹, R¹⁰ et R¹³ représentent chacun indépendamment un atome d'hydrogène ou un groupe méthyle,
X est un chloro, et
Y est un atome d'hydrogène; ou
leurs énantiomères optiques.

7. Dans des tests employant des cellules végétales, des extraits cellulaires ou de la tubuline, procédés selon la revendication 3 utilisant des amides d'acide benzoïque ayant la formule développée (II) dans laquelle
R¹ est un groupe méthyle,
R² est un groupe méthyle ou éthyle,
R³ et R⁵ représentent chacun indépendamment un atome d'hydrogène, un halogéno, un groupe méthyle, nitro ou cyano, pourvu que R³ et R⁵ ne soient pas tous deux des atomes d'hydrogène,
R⁴ est un atome d'hydrogène,
X est un chloro, et
Y est un atome d'hydrogène; ou
leurs énantiomères optiques.

8. Procédés selon la revendication 1 utilisant des pyridinecarboxamides ayant la formule développée suivante 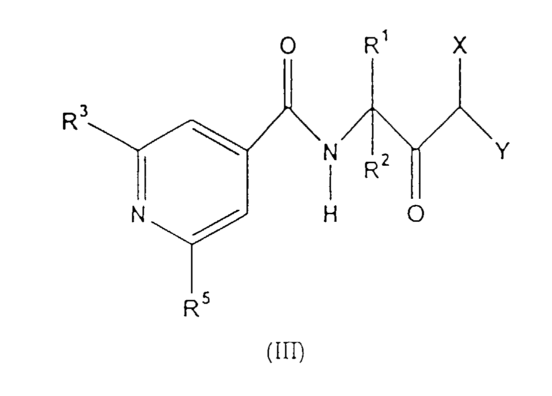 dans laquelle
R¹ et R² représentent chacun indépendamment un atome d'hydrogène, un groupe alkyle en C₁-C₆, halogénoalkyle en C₁-C₆, alcényle en C₂-C₆, ou alcynyle en C₂-C₆, pourvu que R¹ et R² ne soient pas tous deux des atomes d'hydrogène;
R³ et R⁵ représentent chacun indépendamment un atome d'hydrogène, un halogéno, un groupe cyano, alkyle en C₁-C₆, halogénoalkyle en C₁-C₆, alcényle en C₂-C₆, alcynyle en C₂-C₆, alcoxy en C₁-C₆, alkylthio en C₁-C₆, halogénoalcoxy en C₁-C₆, nitro, carboxy, -NR⁶R⁷, -CR⁸=NOR⁹, -NHCOOR¹⁰, -CONR¹¹R¹² ou -COOR¹³;
R⁶, R⁷, R⁸, R⁹, R¹⁰, R¹¹, R¹² et R¹³ représentent un atome d'hydrogène ou un groupe alkyle en C₁-C₆; et
X et Y représentent chacun indépendamment un atome d'hydrogène, un halogéno, un groupe cyano, thiocyano, isothiocyano ou alkyl(en C₁-C₆)sulfonyloxy, pourvu que X et Y ne soient pas tous deux des atomes d'hydrogène; ou
leurs énantiomères optiques.

9. Procédés selon la revendication 1 utilisant des pyridinecarboxamides ayant la formule développée suivante 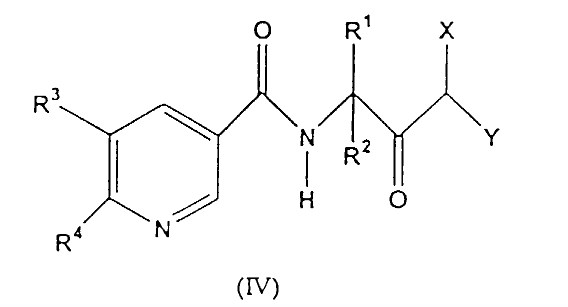 dans laquelle
R¹ et R² représentent chacun indépendamment un atome d'hydrogène, un groupe allyle en C₁-C₆, halogénoalkyle en C₁-C₆, alcényle en C₁-C₆ ou alcynyle en C₁-C₆, pourvu que R¹ et R² ne soient pas tous deux des atomes d'hydrogène;
R¹ et R⁴ représentent chacun indépendamment un atome d'hydrogène, un halogéno, un groupe cyano, alkyle en C₁-C₆, halogénoalkyle en C₁-C₆, alcényle en C₂-C₆, alcynyle en C₂-C₆, alcoxy en C₁-C₆, alkylthio en C₁-C₆, halogénoalcoxy en C₁-C₆, nitro, carboxy, -NR⁶R⁷, -CR⁸=NOR⁹, -NHCOOR¹⁰, -CONR¹¹R¹² ou -COOR¹³; ou R³ et R⁴ peuvent former ensemble un noyau condensé à 5, 6 ou 7 chaînons qui peut contenir jusqu'à deux hétéroatomes choisis dans le groupe constitué par O, S, N et P;
R⁶, R⁷, R⁸, R⁹, R¹⁰, R¹¹, R¹² et R¹³ représentent chacun indépendamment un atome d'hydrogène ou un groupe alkyle en C₁-C₆; et
X et Y représentent chacun indépendamment un atome d'hydrogène, un halogéno, un groupe cyano, thiocyano, isothiocyano ou alkyl(en C₁-C₆)sulfonyloxy, pourvu que X et Y ne soient pas tous deux des atomes d'hydrogène; ou
leurs énantiomères optiques.

10. Procédés selon la revendication 9 utilisant des pyridinecarboxamides ayant la formule développée (IV) dans laquelle
R¹ est un groupe méthyle;
R² est un groupe méthyle ou éthyle;
R³ et R⁴ représentent chacun indépendamment un atome d'hydrogène, un halogéno, un groupe cyano, méthyle, nitro, -NR⁶R⁷, -CR⁸=NOR⁹ ou -NHCOOR¹⁰, pourvu que R³ et R⁴ ne pas tous deux des atomes d'hydrogène;
R⁶, R⁷, R⁸, R⁹, R¹⁰ et R¹³ représentent chacun indépendamment un atome d'hydrogène ou un groupe allyle en C₁-C₆;
X est un chloro et Y est un atome d'hydrogène; ou
leurs énantiomères optiques.

11. Procédés selon la revendication 1 utilisant des furylcarboxamides ou des thiénylcarboxamides ayant la formule développée suivante: 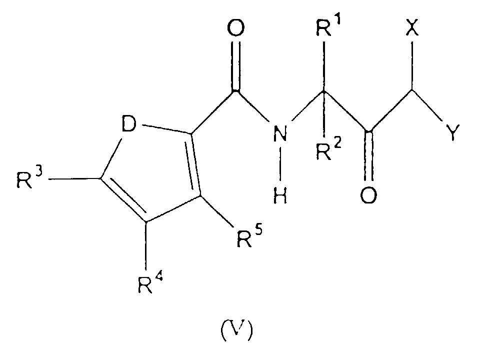 dans laquelle
D est O ou S;
R¹ et R² représentent chacun indépendamment un atonie d'hydrogène, un groupe alkyle en C₁-C₆, halogénoalkyle en C₁-C₆, alcényle en C₂-C₆, et alcynyle en C₂-C₆, pourvu que R¹ et R² ne soient pas tous deux des atomes d'hydrogène;
R³, R⁴, et R⁵ représentent chacun indépendamment un atome d'hydrogène, un halogéno, un groupe cyano, alkyle en C₁-C₆, halogénoalkyle en C₁-C₆, alcényle en C₂-C₆, alcynyle en C₂-C₆, alcoxy en C₁-C₆, alkylthio en C₁-C₆, halogénoalcoxy en C₁-C₆, nitro, carboxy, -NR⁶R⁷, -CR⁸=NOR⁹, -NHCOOR¹⁰, -CONR¹¹R¹² ou -COOR¹³; ou R³ et R⁴ peuvent former ensemble un noyau condensé à 5, 6 ou 7 chaînons qui peut contenir jusqu'à deux hétéroatomes choisis dans le groupe constitué par O, S, N et P;
R⁶, R⁷, R⁸, R⁹, R¹⁰, R¹¹, R¹² et R¹³ représentent chacun indépendamment un atome d'hydrogène ou un groupe alkyle en C₁-C₆; et
X et Y représentent chacun indépendamment un atome d'hydrogène, un halogéno, un groupe cyano, thiocyano, isothiocyano ou alkyl(en C₁-C₆)sulfonyloxy, pourvu que X et Y ne soient pas tous deux des atomes d'hydrogène; ou
leurs énantiomères optiques.

12. Procédés selon la revendication 1 utilisant des furylcarboxamides ou des thiénylcarboxamides ayant la formule développée suivante: 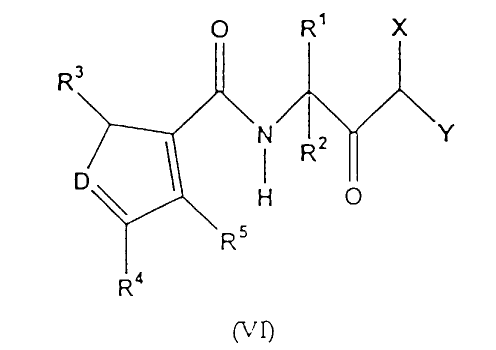 dans laquelle
D est O ou S;
R¹ et R² représentent chacun indépendamment un atome d'hydrogène, un groupe alkyle en C₁-C₆, halogénoalkyle en C₁-C₆, alcényle en C₂-C₆, et alcynyle en C₂-C₆, pourvu que R' et R² ne pas tous deux des atomes d'hydrogène;
R³, R⁴ et R⁵ représentent chacun indépendamment un atome d'hydrogène, un halogéno, un groupe cyano, alkyle en C₁-C₆, halogénoalkyle en C₁-C₆, alcényle en C₂-C₆, alcynyle en C₂-C₆, alcoxy en C₁-C₆, alkylthio en C₁-C₆, halogénoalcoxy en C₁-C₆, nitro, carboxy, -NR⁶R⁷, -CR⁸=NOR⁹, -NHCOOR¹⁰, -CONR¹¹R¹² ou -COOR¹³;
R⁶, R⁷, R⁸, R⁹, R¹⁰, R¹¹, R¹² et R¹³ représentent chacun indépendamment un atome d'hydrogène ou un groupe alkyle en C₁-C₆; et
X et Y sont choisis indépendamment parmi un atome d'hydrogène, un halogéno, un groupe cyano, thiocyano, isothiocyano et alkyl(en C₁-C₆)sulfonyloxy, pourvu que X et Y ne soient pas tous deux des atomes d'hydrogène; ou
leurs énantiomères optiques.
